# EUROPEAN PATENT SPECIFICATION

(11) **EP 2 032 531 B1**
(45) Date of publication and mention of the grant of the patent: **10.02.2016**
(21) Application number: 07784348.0
(22) Date of filing: 07.06.2007
(51) Int. Cl.: C07D 209/14, A61K 31/4045, A61P 35/00

(54) **SALTS OF N-HYDROXY-3-[4-[[[2-(2-METHYL-1H-INDOL-3-YL)ETHYL]AMINO]METHYL]PHENYL]-2E-2-PROPENAMIDE**
SALZE AUS N-HYDROXY-3-[4-[[[2-(2-METHYL-1H-INDOL-3-YL)ETHYL]AMINO]METHYL]PHENYL]-2E-2-PROPENAMID
SELS DE N-HYDROXY-3-[4-[[[2-(2-MÉTHYL-1H-INDOL-3-YL)ÉTHYL]AMINO]MÉTHYL]PHÉNYL]-2E-2-PROPÉNAMIDE

(30) Priority: 12.06.2006 US 804523 P; 14.12.2006 US 869993 P
(43) Date of publication of application: 11.03.2009
(73) Proprietor: Novartis AG, 4056 Basel (CH)
(72) Inventor: ACEMOGLU, Murat, CH-4052 Basel (CH); BAJWA, Joginder, S., Elmwood Park, New Jersey 07407 (US); KARPINSKI, Piotr, Lincoln Park, New Jersey 07035 (US); PAPOUTSAKIS, Dimitris, Acton, Massachusetts 01720 (US); SLADE, Joel, Flanders, New Jersey 07836 (US); STOWASSER, Frank, 79730 Murg (DE)
(74) Representative: Roth, Peter Richard
(86) International application number: PCT/US2007/070558
(87) International publication number: WO 2007/146715

(56) References cited:
- WO-A-02/22577
- WO-A-2005/013958

## Description

### Field of the Invention

This invention relates to a salt of N-hydroxy-3-[4-[[[2-(2-methyl-1H-indol-3-yl)ethyl]amino]methyl]phenyl]-2E-2-propenamide, as well as to pharmaceutical compositions comprising the same. Methods of treatment using the same are also described herein.

### Related Background Art

The compound N-hydroxy-3-[4-[[[2-(2-methyl-1H-indol-3-yl)ethyl]amino]methyl]phenyl]-2E-2-propenamide (alternatively, N-hydroxy-3-(4-{[2-(2-methyl-1H-indol-3-yl)-ethylamino]-methyl}-phenyl)-acrylamide) has the formula (I): 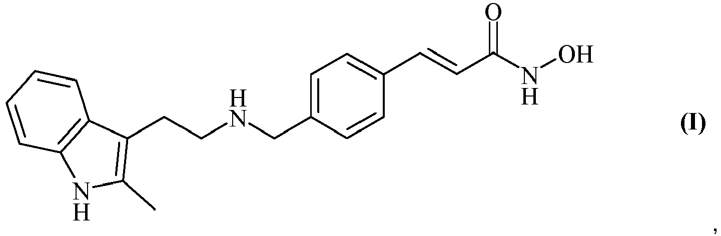 as described in WO 02/22577. Valuable pharmacological properties are attributed to this compound; thus, it can be used, for example, as a histone deacetylase inhibitor useful in therapy for diseases which respond to inhibition of histone deacetylase activity. WO 02/22577 does not disclose any specific salts or salt hydrates or solvates of N-hydroxy-3-[4-[[[2-(2-methyl-1H-indol-3-yl)ethyl]amino]methyl]phenyl]-2E-2-propenamide.

WO2005/013958 describes histone deacetylase inhibitor compounds which include the compound of formula (I) above as immunosuppressant agents. Pharmaceutically acceptable salts of such histone deacetylase inhibitor compounds are generally mentioned in WO2005/013958.

### Summary of the Invention

The present invention is directed to the anhydrous lactate salt of N-hydroxy-3-[4-[[[2-(2-methyl-1H-indol-3-yl)ethyl]amino]methyl]phenyl]-2E-2-propenamide.

The invention is further directed to pharmaceutical compositions comprising (a) a therapeutically effective amount of an anhydrous lactate salt of N-hydroxy-3-[4-[[[2-(2-methyl-1H-indol-3-yl)ethyl]amino]methyl]phenyl]-2E-2-propenamide; and (b) at least one pharmaceutically acceptable carrier, diluent, vehicle or excipient, as described in the claims.

A compound for use in a method of treating a disease which responds to an inhibition of histone deacetylase activity comprising the step of administering to a subject in need of such treatment a therapeutically effective amount of an inventive salt of N-hydroxy-3-[4-[[[2-(2-methyl-1H-indol-3-yl)ethyl]amino]methyl]phenyl]-2E-2-propenamide is also described herein.

### Brief Description of the Drawings

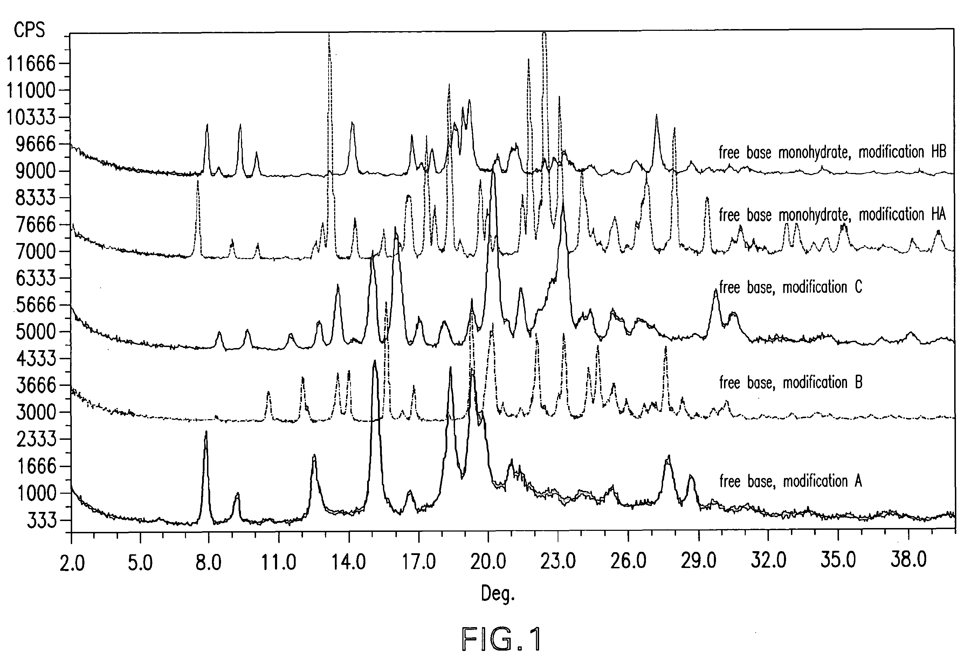

Figure 1 shows the x-ray powder diffraction patterns for forms A, B, C, H_{A} and H_{B} of N-hydroxy-3-[4-[[[2-(2-methyl-1H-indol-3-yl)ethyl]amino]methyl]phenyl]-2E-2-propenamide free base.
Figure 2 shows the x-ray powder diffraction pattern for the hydrochloride salt of N-hydroxy-3-[4-[[[2-(2-methyl-1H-indol-3-yl)ethyl]amino]methyl]phenyl]-2E-2-propenamide.
Figures 3A, 3B and 3C show the x-ray powder diffraction patterns for forms A, H_{A} and S_{A}, respectively, of the DL-lactate salt of N-hydroxy-3-[4-[[[2-(2-methyl-1H-indol-3-yl)ethyl]amino]methyl]phenyl]-2E-2-propenamide. Figures 3D and 3E show the x-ray powder diffraction patterns for the anhydrous L-lactate and D-lactate salts, respectively, of N-hydroxy-3-[4-[[[2-(2-methyl-1H-indol-3-yl)ethyl]amino]methyl]phenyl]-2E-2-propenamide.
Figure 4 shows the x-ray powder diffraction patterns for forms A and H_{A} of the maleate salt of N-hydroxy-3-[4-[[[2-(2-methyl-1H-indol-3-yl)ethyl]amino]methyl]phenyl]-2E-2-propenamide.
Figure 5 shows the x-ray powder diffraction patterns for forms A, B and C of the hemi-tartarate salt of N-hydroxy-3-[4-[[[2-(2-methyl-1H-indol-3-yl)ethyl]amino]methyl]phenyl]-2E-2-propenamide.
Figure 6 shows the x-ray powder diffraction patterns for forms A and B of the mesylate (methanesulfonate) salt of N-hydroxy-3-[4-[[[2-(2-methyl-1H-indol-3-yl)ethyl]amino]methyl]phenyl]-2E-2-propenamide.
Figure 7 shows the x-ray powder diffraction patterns for forms A and S_{A} of the acetate salt of N-hydroxy-3-[4-[[[2-(2-methyl-1H-indol-3-yl)ethyl]amino]methyl]phenyl]-2E-2-propenamide.
Figure 8 shows the x-ray powder diffraction patterns for forms A, S_{A} and S_{B} of the benzoate salt of N-hydroxy-3-[4-[[[2-(2-methyl-1H-indol-3-yl)ethyl]amino]methyl]phenyl]-2E-2-propenamide according to the present invention.
Figure 9 shows the x-ray powder diffraction patterns for the citrate salt of N-hydroxy-3-[4-[[[2-(2-methyl-1H-indol-3-yl)ethyl]amino]methyl]phenyl]-2E-2-propenamide according to the present invention.
Figure 10 shows the x-ray powder diffraction patterns for forms A, B and H_{A} of the hemi-fumarate salt of N-hydroxy-3-[4-[[[2-(2-methyl-1H-indol-3-yl)ethyl]amino]methyl]phenyl]-2E-2-propenamide.
Figure 11 shows the x-ray powder diffraction patterns for the gentisate salt of N-hydroxy-3-[4-[[[2-(2-methyl-1H-indol-3-yl)ethyl]amino]methyl]phenyl]-2E-2-propenamide according to the present invention.
Figure 12 shows the x-ray powder diffraction patterns for forms A and S_{A} of the hemi-malate salt of N-hydroxy-3-[4-[[[2-(2-methyl-1H-indol-3-yl)ethyl]amino]methyl]phenyl]-2E-2-propenamide according to the present invention.
Figure 13 shows the x-ray powder diffraction patterns for the malonate salt of N-hydroxy-3-[4-[[[2-(2-methyl-1H-indol-3-yl)ethyl]amino]methyl]phenyl]-2E-2-propenamide according to the present invention.
Figure 14 shows the x-ray powder diffraction patterns for the oxalate salt of N-hydroxy-3-[4-[[[2-(2-methyl-1H-indol-3-yl)ethyl]amino]methyl]phenyl]-2E-2-propenamide according to the present invention.
Figure 15 shows the x-ray powder diffraction patterns for forms A, S_{A}, S_{B} and H_{A} of the phosphate salt of N-hydroxy-3-[4-[[[2-(2-methyl-1H-indol-3-yl)ethyl]amino]methyl]phenyl]-2E-2-propenamide.
Figure 16 shows the x-ray powder diffraction patterns for forms A and S_{A} of the propionate salt of N-hydroxy-3-[4-[[[2-(2-methyl-1H-indol-3-yl)ethyl]amino]methyl]phenyl]-2E-2-propenamide.
Figure 17 shows the x-ray powder diffraction patterns for forms A and S_{A} of the sulfate salt of N-hydroxy-3-[4-[[[2-(2-methyl-1H-indol-3-yl)ethyl]amino]methyl]phenyl]-2E-2-propenamide.
Figure 18 shows the x-ray powder diffraction patterns for forms A, B, S_{A} and H_{A} of the hemi-succinate salt of N-hydroxy-3-[4-[[[2-(2-methyl-1H-indol-3-yl)ethyl]amino]methyl]phenyl]-2E-2-propenamide.

### Detailed Description of the Invention

As used herein, "salt" refers to a compound prepared by the reaction of an organic acid or base drug with a pharmaceutically acceptable mineral or organic acid or base; as used herein, "salt" includes hydrates and solvates of salts made in accordance with this invention. Exemplary pharmaceutically acceptable mineral or organic acids or bases are as listed in Tables 1-8 in Handbook of Pharmaceutical Salts, P.H. Stahl and C.G. Wermuth (eds.), VHCA, Zurich 2002, pp. 334-345. As used herein, "polymorph" refers to a distinct "crystal modification" or "polymorphic form" or "crystalline form", which differs from another with respect to x-ray powder diffraction pattern, physicochemical and/or pharmacokinetic properties, and thermodynamic stability.

The present invention provides the anhydrous lactate salt of N-hydroxy-3-[4-[[[2-(2-methyl-1H-indol-3-yl)ethyl]amino]methyl]phenyl]-2E-2-propenamide. The present invention provides the anhydrous lactate (DL-lactate, L-lactate, D-lactate forms) salts of N-hydroxy-3-[4-[[[2-(2-methyl-1H-indol-3-yl)ethyl]amino]methyl]phenyl]-2E-2-propenamide.

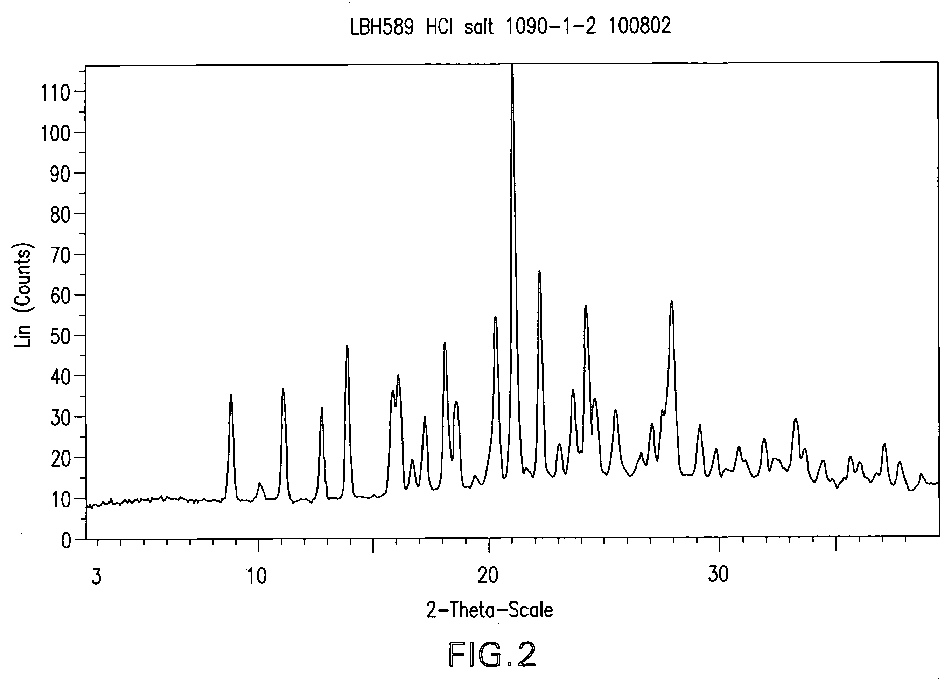

There is also described the hydrochloride salt of N-hydroxy-3-[4-[[[2-(2-methyl-1H-indol-3-yl)ethyl]amino]methyl]phenyl]-2E-2-propenamide, preferably the 1:1 hydrochloride salt of N-hydroxy-3-[4-[[[2-(2-methyl-1H-indol-3-yl)ethyl]amino]methyl]phenyl]-2E-2-propenamide. The hydrochloride salt has a good aqueous solubility of 2.4 mg/mL and a good intrinsic dissolution rate. It also shows high solubility in methanol and considerable solubility in other common organic solvents. It is produced as a single, excellently crystalline, anhydrous/unsolvated polymorph with a decomposition temperature of about 235.7°C. It is non-hygroscopic (0.32%) and is the prevailing form of N-hydroxy-3-[4-[[[2-(2-methyl-1H-indol-3-yl)ethyl]amino]methyl]phenyl]-2E-2-propenamide in the presence of the chloride ion at high concentrations. No additional polymorphs are detected upon equilibration at ambient temperature; the hydrochloride salt converts to the free base in a phosphate buffer (pH = 6.8). The XRPD of the hydrochloride salt of N-hydroxy-3-[4-[[[2-(2-methyl-1H-indol-3-yl)ethyl]amino]methyl]phenyl]-2E-2-propenamide is shown in Figure 2.

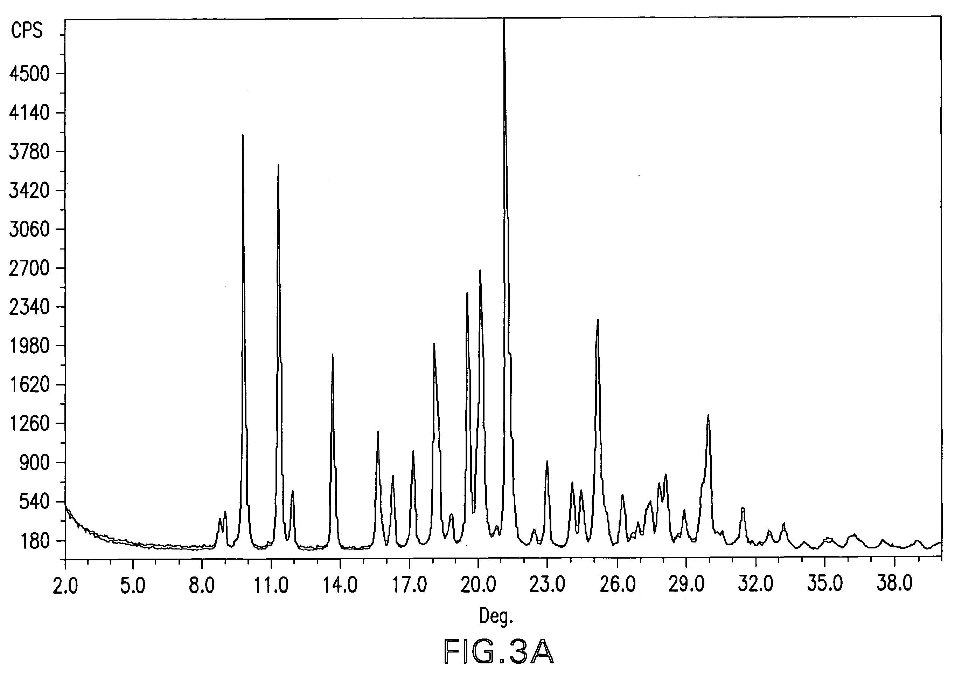

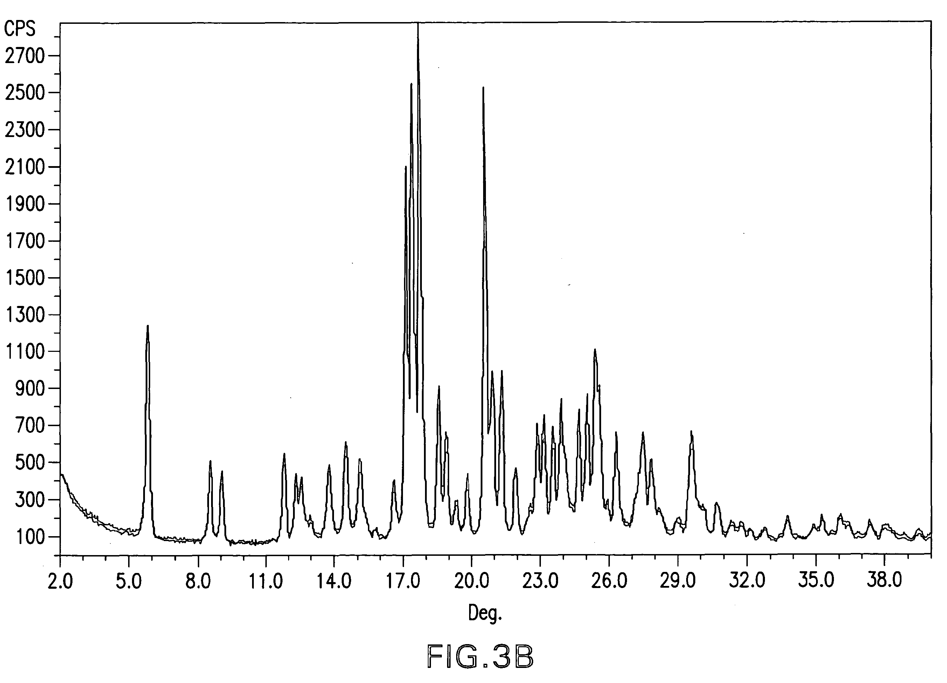

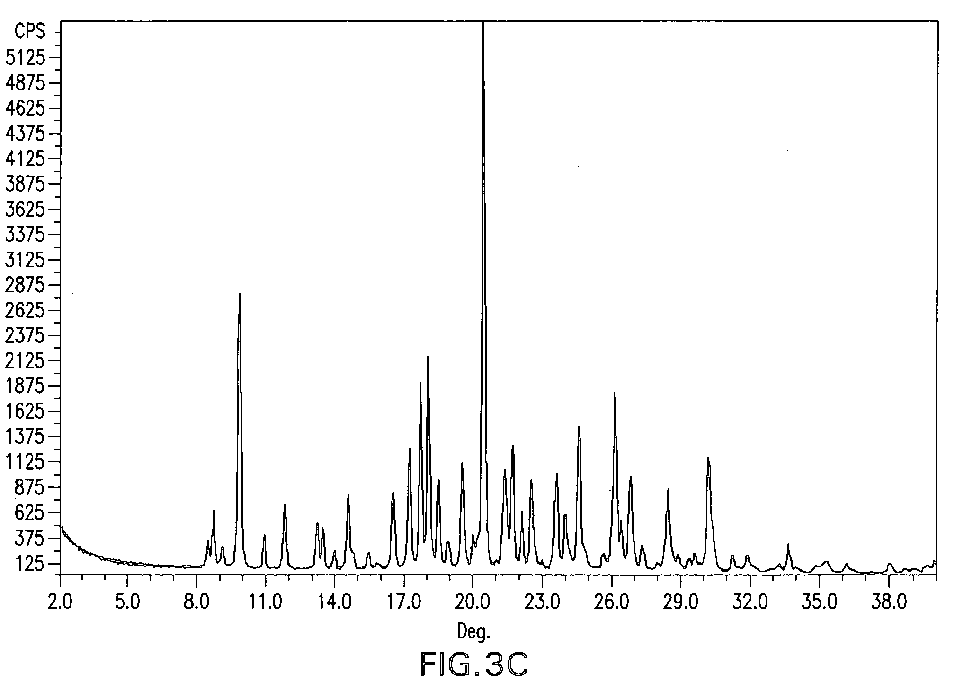

The present invention is further directed to an anhydrous lactate salt of N-hydroxy-3-[4-[[[2-(2-methyl-1H-indol-3-yl)ethyl]amino]methyl]phenyl]-2E-2-propenamide. The invention also provides the 1:1 anhydrous DL-lactate salt of N-hydroxy-3-[4-[[[2-(2-methyl-1H-indol-3-yl)ethyl]amino]methyl]phenyl]-2E-2-propenamide. Polymorphic forms A, H_{A} and S_{A} for the DL-lactate salt can be seen in the XRPD patterns shown in Figures 3A-3C, respectively. The DL-lactate salt has an excellent aqueous solubility and a good intrinsic dissolution. Polymorphic form A of the DL-lactate salt (anhydrous DL-lactate salt) melts and decomposes at around 183-186°C and is slightly hygroscopic with a loss on drying (LOD) of 0.2% until 120°C. Form A is more stable in organic solvents and in water than the other forms of the DL-lactate salt. Under most circumstances, form A does not convert into any other form, though upon equilibration at pH 1 and 2, the chloride salt is formed and at 0°C and 10°C and in acetone/water mixture, form A was observed along with form H_{A} of the DL-lactate salt. Form H_{A} of the DL-lactate salt (monohydrate DL-lactate salt) melts and decomposes at around 120°C and is slightly hygroscopic with a LOD of 0.4% until 110°C, 3.0% until 130°C and 4.4% until 155°C (with degradation). Under most circumstances, form H_{A} slowly converts into form A, though upon equilibration at pH 1 and 2, the chloride salt is formed. Upon equilibration in methanol, form H_{A} of the DL-lactate salt converts to form S_{A} which is a monomethanol solvate of the DL-lactate salt. Form S_{A} melts and decomposes at around 123°C with a LOD of 5.9% until 140°C (with degradation).

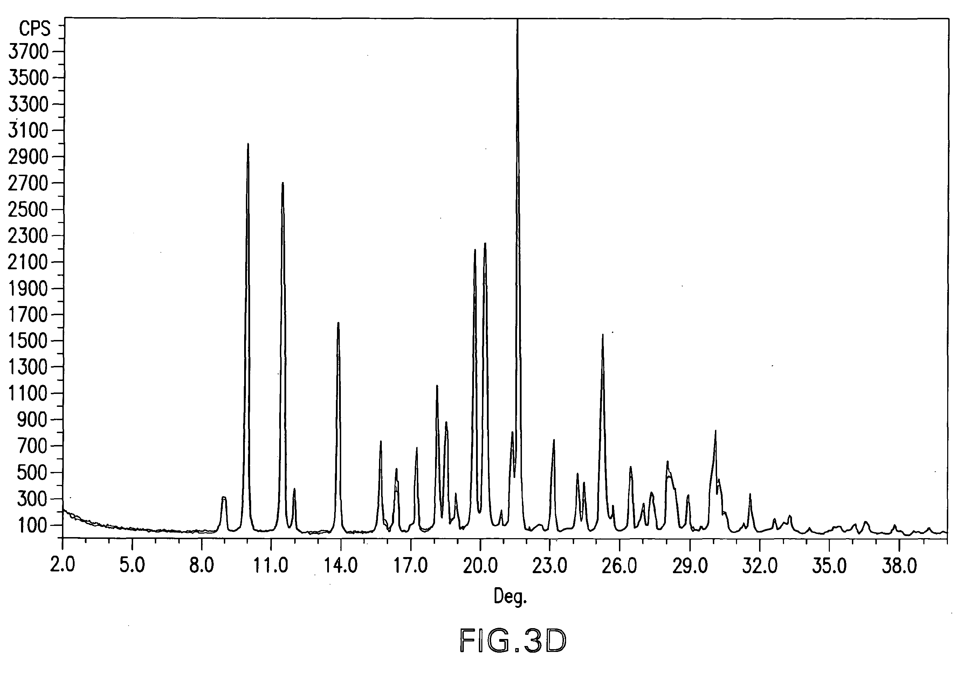

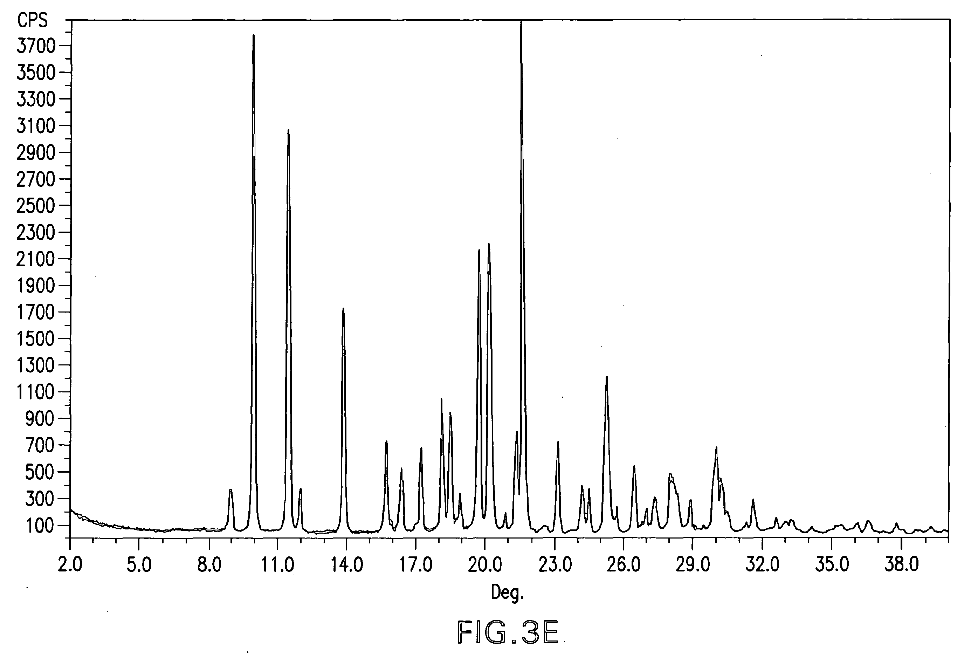

The present invention also provides the anhydrous L-(+)-lactate salt of N-hydroxy-3-[4-[[[2-(2-methyl-1H-indol-3-yl)ethyl]amino]methyl]phenyl]-2E-2-propenamide. The XRPD pattern for the L-(+)-lactate salt of N-hydroxy-3-[4-[[[2-(2-methyl-1H-indol-3-yl)ethyl]amino]methyl]phenyl]-2E-2-propenamide is shown in Figure 3D. Melting and decomposition both take place at around 184.7°C for the L-(+)-lactate salt of N-hydroxy-3-[4-[[[2-(2-methyl-1H-indol-3-yl)ethyl]amino]methyl]phenyl]-2E-2-propenamide anhydrate form. The present invention also provides the anhydrous D-(-)-lactate salt of N-hydroxy-3-[4-[[[2-(2-methyl-1H-indol-3-yl)ethyl]amino]methyl]phenyl]-2E-2-propenamide. The XRPD pattern for the D-(-)-lactate salt of N-hydroxy-3-[4-[[[2-(2-methyl-1H-indol-3-yl)ethyl]amino]methyl]phenyl]-2E-2-propenamide is shown in Figure 3E. Melting and decomposition both take place at around 184.1°C for the D-(-)-lactate salt of N-hydroxy-3-[4-[[[2-(2-methyl-1H-indol-3-yl)ethyl]amino]methyl]phenyl]-2E-2-propenamide anhydrate form.

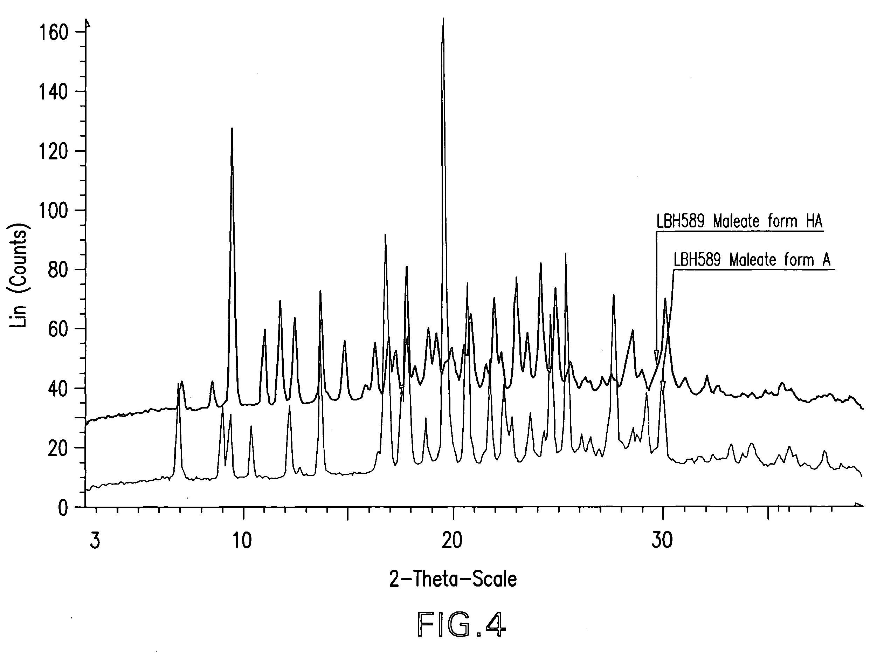

The present disclosure also provides the maleate salt of N-hydroxy-3-[4-[[[2-(2-methyl-1H-indol-3-yl)ethyl]amino]methyl]phenyl]-2E-2-propenamide, preferably the 1:1 maleate salt of N-hydroxy-3-[4-[[[2-(2-methyl-1H-indol-3-yl)ethyl]amino]methyl]phenyl]-2E-2-propenamide. Maleic acid is the only dicarboxylic acid salt forming agent which forms a 1:1 salt with N-hydroxy-3-[4-[[[2-(2-methyl-1H-indol-3-yl)ethyl]amino]methyl]phenyl]-2E-2-propenamide. Polymorphic forms A and H_{A} for the maleate salt can be seen in the XRPD patterns shown in Figure 4. Form A of the maleate salt, upon heating, decomposes without melting at around 177°C. Its LOD is less than 0.2% at 150°C, and it is non hygroscopic. The maleate salt has a good aqueous solubility of 2.6 mg/mL and a good intrinsic dissolution. It shows high solubility in methanol and ethanol and considerable solubility in other common organic solvents. Form H_{A} of the maleate salt, a hydrate of form A, upon heating, decomposes without melting at around 150°C. LOD is around 6.0% at 100°C.

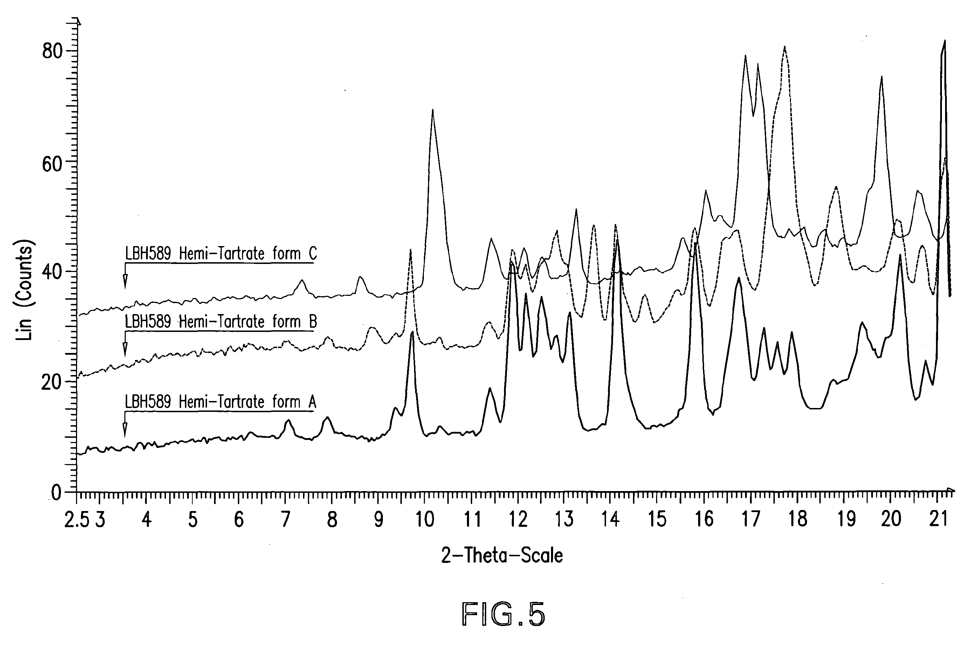

The present disclosure is further directed to the mesylate (or methanesulfonate) salt of N-hydroxy-3-[4-[[[2-(2-methyl-1H-indol-3-yl)ethyl]amino]methyl]phenyl]-2E-2-propenamide, preferably the 1:1 mesylate salt of N-hydroxy-3-[4-[[[2-(2-methyl-1H-indol-3-yl)ethyl]amino]methyl]phenyl]-2E-2-propenamide. Forms A and B for the mesylate salt can be seen in the XRPD patterns shown in Figure 5. Form A of the mesylate salt upon heating, decomposes without melting at around 192°C. Its LOD is less than 0.2% at 150°C, and form A is very slightly hygroscopic (less than 0.35% moisture at 85% r.h.). The mesylate salt has an excellent aqueous solubility of 12.9 mg/mL and a high intrinsic dissolution rate. It has high solubility in methanol and ethanol and appreciable solubility in the remaining organic solvents. Upon equilibration, form A converts to form B in water, to the hydrochloride salt in 0.1 N HCl, and to the free base in a phosphate buffer (pH = 6.8). Form B of the mesylate salt can by obtained from reaction in ethyl acetate at ambient temperature, with subsequent heating of the suspension to 50°C or from the conversion of form A in water. The mesylate salt is isolated in at least four crystalline modifications, two of which are highly crystalline, slightly hygroscopic (0.82%), white solids (including forms A and B) and the other two of which were yellow in color and contained more than the stoichiometrical excess of methanesulfonic acid, i.e., less than a half mol additional per mol of N-hydroxy-3-[4-[[[2-(2-methyl-1H-indol-3-yl)ethyl]amino]methyl]phenyl]-2E-2-propenamide; the latter two forms are highly hygroscopic, i.e., weight gain of at least ∼40% at 93% r.h.

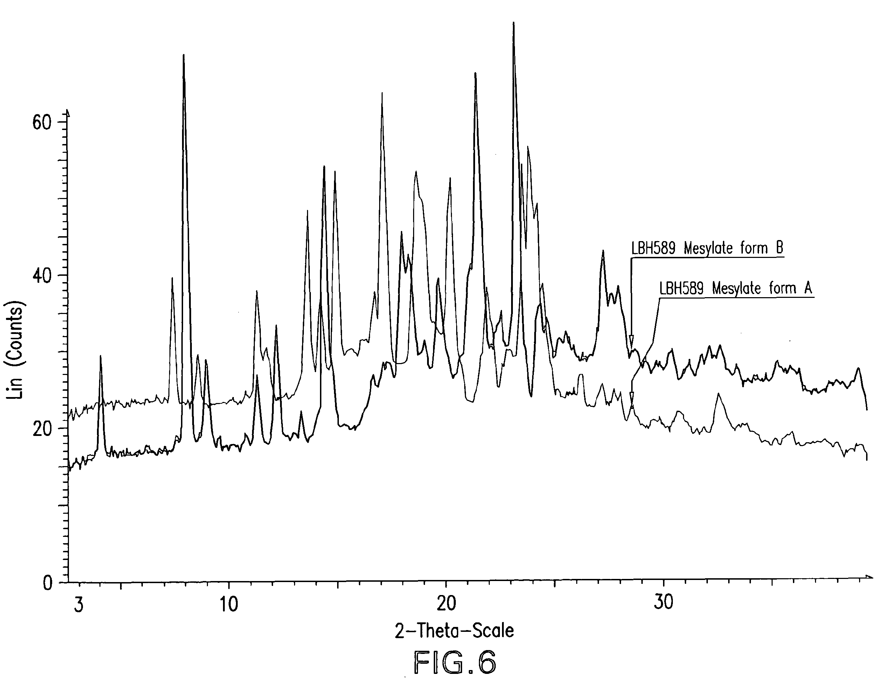

The present disclosure is further directed to the tartrate salt of N-hydroxy-3-[4-[[[2-(2-methyl-1H-indol-3-yl)ethyl]amino]methyl]phenyl]-2E-2-propenamide, preferably the 2:1 tartrate (hemi-tartrate) salt of N-hydroxy-3-[4-[[[2-(2-methyl-1H-indol-3-yl)ethyl]amino]methyl]phenyl]-2E-2-propenamide, and more preferably the 2:1 L-tartrate salt of N-hydroxy-3-[4-[[[2-(2-methyl-1H-indol-3-yl)ethyl]amino]methyl]phenyl]-2E-2-propenamide. Forms A, B and C for the hemi-tartrate salt can be seen in the XRPD patterns shown in Figure 6. Form A of the L-tartrate salt, an anhydrous hemi-tartrate, upon heating, decomposes without melting at around 209°C. LOD is less than 0.3% at 150°C, and form A is slightly hygroscopic (less than 0.5% moisture at 85% r.h.). The L-tartrate salt has a good aqueous solubility of 3.5 mg/mL and a good intrinsic dissolution. It shows good solubility in acetone, ethyl acetate and other common organic solvents and limited solubility in alcohols. Upon equilibration, form A converts to form C in methanol, to the hydrochloride salt in 0.1 N HCl, and to the free base in a phosphate buffer (pH = 6.8). Form B of the tartrate salt, also an anhydrous hemi-tartrate, upon heating, decomposes without melting above 160°C. LOD is less than 2.0% at 150°C, indicating its hygroscopic nature. Form C of the tartrate salt is obtained from equilibration of form A in acetone at ambient temperature.

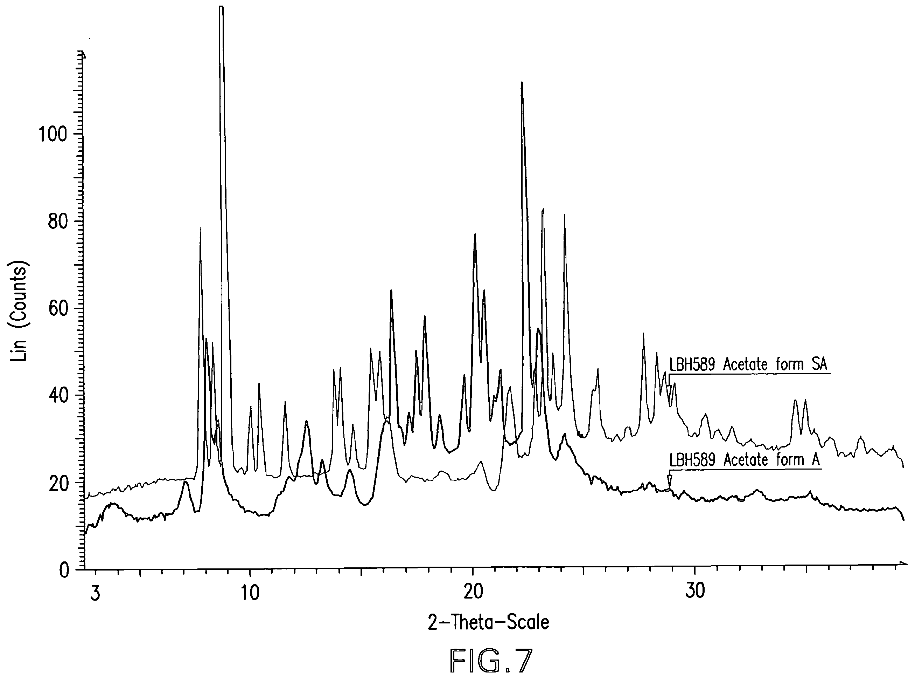

The present disclosure is further directed to the acetate salt of N-hydroxy-3-[4-[[[2-(2-methyl-1H-indol-3-yl)ethyl]amino]methyl]phenyl]-2E-2-propenamide, preferably the 1:1 acetate salt of N-hydroxy-3-[4-[[[2-(2-methyl-1H-indol-3-yl)ethyl]amino]methyl]phenyl]-2E-2-propenamide. Forms A and S_{A} for the acetate salt can be seen in the XRPD patterns shown in Figure 7. Form A of the acetate salt, upon heating, decomposes quickly without melting above 60°C. It has an approximate aqueous solubility of 2 mg/mL. Form S_{A} of the acetate salt is an acetone solvate with the LOD of 13.5% at around 140°C. This solvate is stable below 90°C.

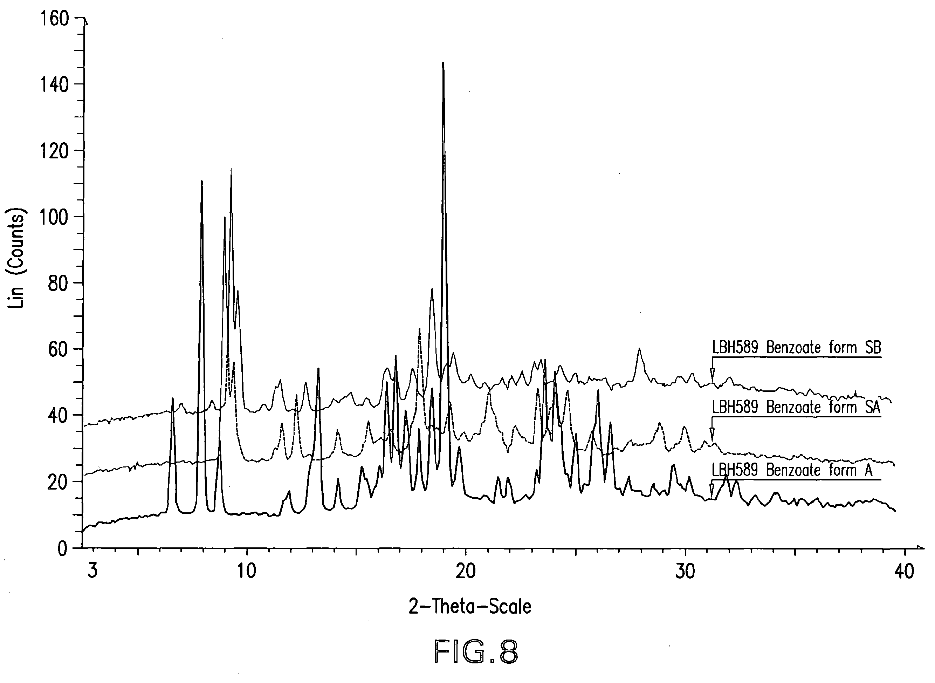

The present disclosure is further directed to the benzoate salt of N-hydroxy-3-[4-[[[2-(2-methyl-1H-indol-3-yl)ethyl]amino]methyl]phenyl]-2E-2-propenamide, preferably the 1:1 benzoate salt of N-hydroxy-3-[4-[[[2-(2-methyl-1H-indol-3-yl)ethyl]amino]methyl]phenyl]-2E-2-propenamide. Forms A, S_{A} and S_{B} for the benzoate salt can be seen in the XRPD patterns shown in Figure 8. Form A of the benzoate salt isolated from reaction in acetone has excellent crystallinity and a high decomposition temperature above 160°C. Its LOD is less than 0.6% at 140°C. It has an approximate aqueous solubility of 0.7 mg/mL. Form S_{A} of the benzoate salt is an ethanol solvate with the LOD of 5.2% before decomposition that occurs above 110°C. Form S_{B} of the benzoate salt is a 2-propanol solvate with the LOD of 6.3% before decomposition that occurs above 100°C.

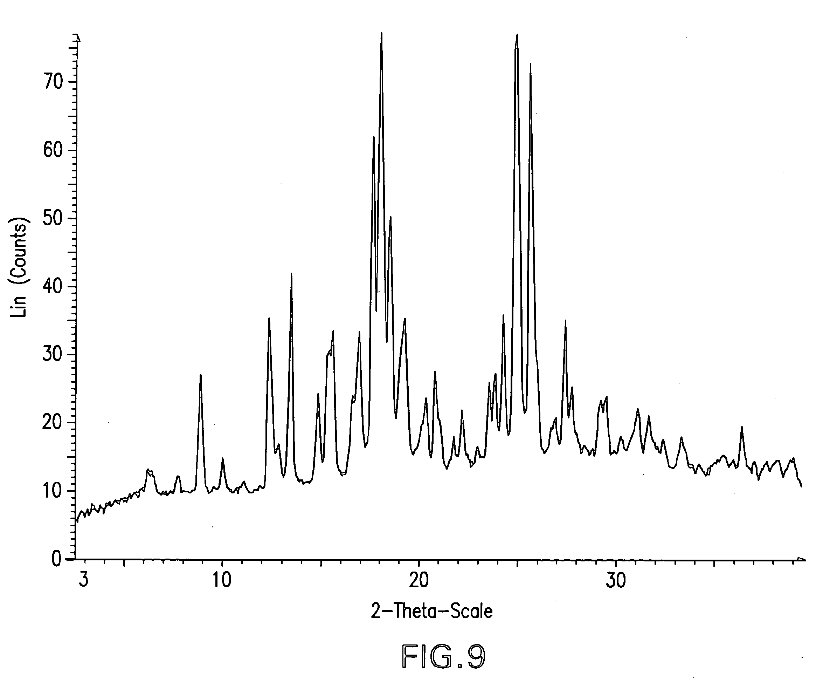

The present disclosure is further directed to the citrate salt of N-hydroxy-3-[4-[[[2-(2-methyl-1H-indol-3-yl)ethyl]amino]methyl]phenyl]-2E-2-propenamide, preferably the 2:1 citrate salt (hemi-citrate) of N-hydroxy-3-[4-[[[2-(2-methyl-1H-indol-3-yl)ethyl]amino]methyl]phenyl]-2E-2-propenamide. The citrate salt can be seen in the XRPD pattern shown in Figure 9. The hemi-citrate salt has an approximate aqueous solubility of 1.2 mg/mL. It is produced as a single, crystalline and anhydrous/unsolvated polymorph with a decomposition temperature above 180°C.

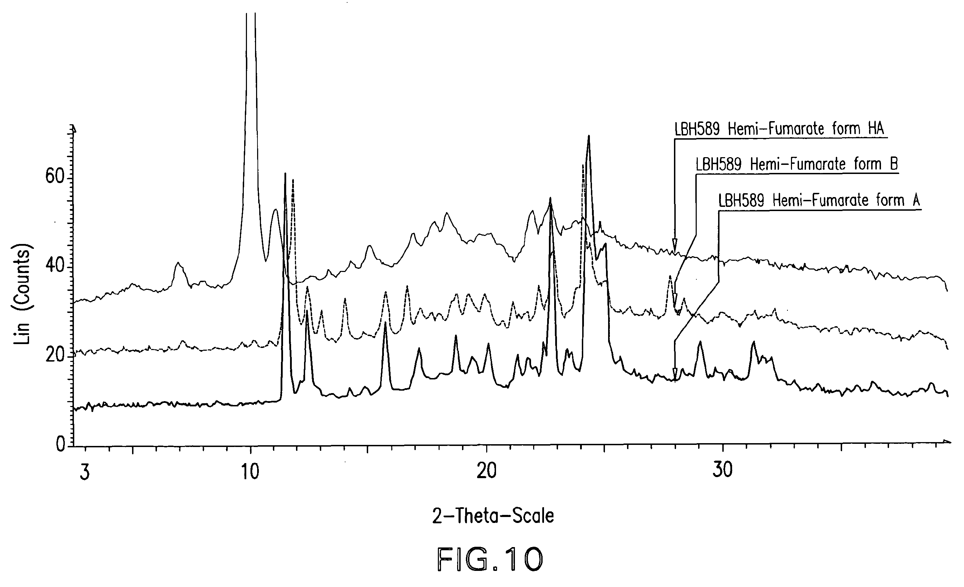

The present disclosure is further directed to the fumarate salt of N-hydroxy-3-[4-[[[2-(2-methyl-1H-indol-3-yl)ethyl]amino]methyl]phenyl]-2E-2-propenamide, preferably the 2:1 fumarate salt (hemi-fumarate) of N-hydroxy-3-[4-[[[2-(2-methyl-1H-indol-3-yl)ethyl]amino]methyl]phenyl]-2E-2-propenamide. Forms A, B, and H_{A} for the hemi-fumarate salt can be seen in the XRPD patterns shown in Figure 10. Form A of the hemi-fumarate salt isolated from reaction in ethanol and water (1:0.05) has excellent crystallinity and a high decomposition temperature of 217°C. Its LOD is less than 0.7% at 200°C. It has an approximate aqueous solubility of 0.4 mg/mL. Form B of the hemi-fumarate salt isolated from reaction in ethanol has good crystallinity and a decomposition temperature above 160°C. It exhibits a two-step LOD: around 1.1% up to 150°C and a subsequent 1.7% between 150°C and 200°C. Form H_{A} of the hemi-fumarate salt, possible hydrate, isolated from reaction in 2-propanol has good crystallinity and decomposition temperature above 100°C. It exhibits a two-step LOD: around 3.5% up to 75°C and a subsequent 6% between 75°C and 150°C.

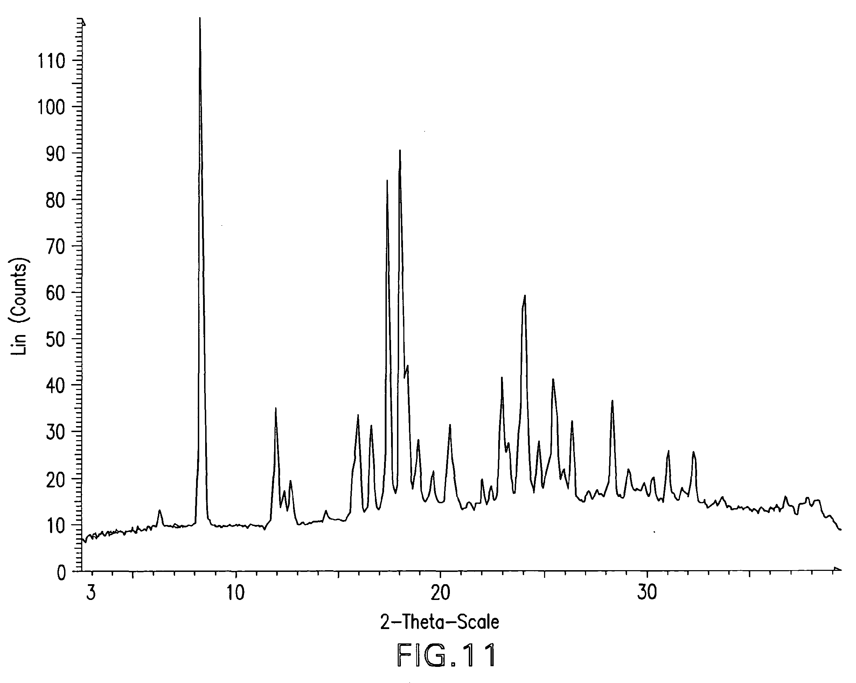

The present disclosure is further directed to the gentisate salt of N-hydroxy-3-[4-[[[2-(2-methyl-1H-indol-3-yl)ethyl]amino]methyl]phenyl]-2E-2-propenamide, preferably the 1:1 gentisate salt of N-hydroxy-3-[4-[[[2-(2-methyl-1H-indol-3-yl)ethyl]amino]methyl]phenyl]-2E-2-propenamide. The gentisate salt can be seen in the XRPD pattern shown in Figure 11. The gentisate salt has an approximate aqueous solubility of 0.3 mg/mL. It is produced as a single, crystalline and anhydrous/unsolvated polymorph.

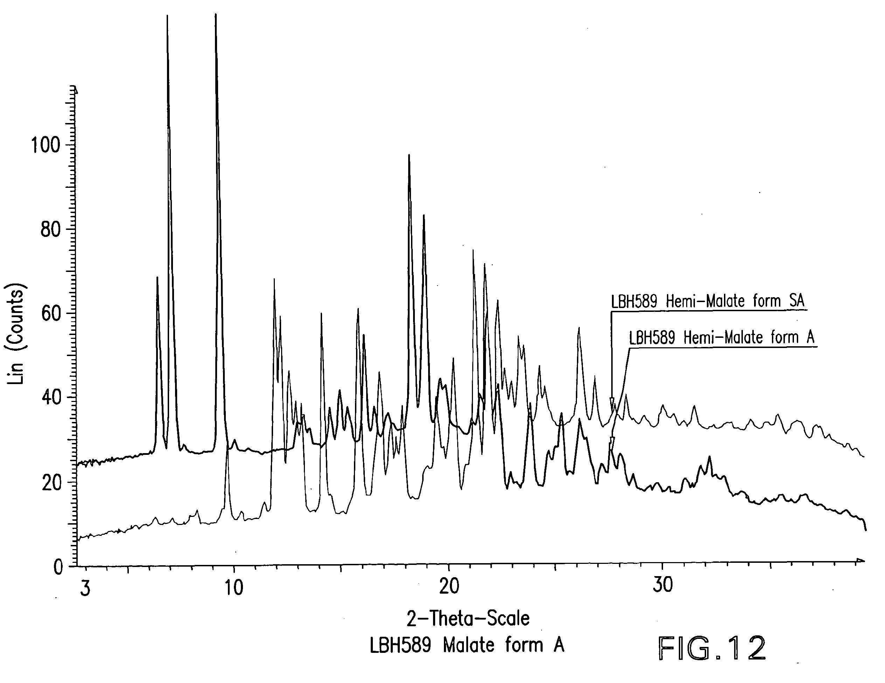

The present disclosure is further directed to the malate salt of N-hydroxy-3-[4-[[[2-(2-methyl-1H-indol-3-yl)ethyl]amino]methyl]phenyl]-2E-2-propenamide, preferably the 2:1 malate (hemi-malate) salt of N-hydroxy-3-[4-[[[2-(2-methyl-1H-indol-3-yl)ethyl]amino]methyl]phenyl]-2E-2-propenamide. Forms A and S_{A} for the hemi-malate salt can be seen in the XRPD patterns shown in Figure 12. Form A of the hemi-malate salt isolated from reaction in ethanol and water (1:0.05) or neat ethanol and 2-propanol, has excellent crystallinity and a high decomposition temperature of 206°C. It exhibits a 2% LOD up to 175°C. It has an approximate aqueous solubility of 1.4 mg/mL. Form S_{A} of the hemi-malate salt was obtained from the salt formation reaction in acetone. It has excellent crystallinity, but decomposes gradually starting at around 80°C. Its LOD up to 75°C amounts to 0.6%.

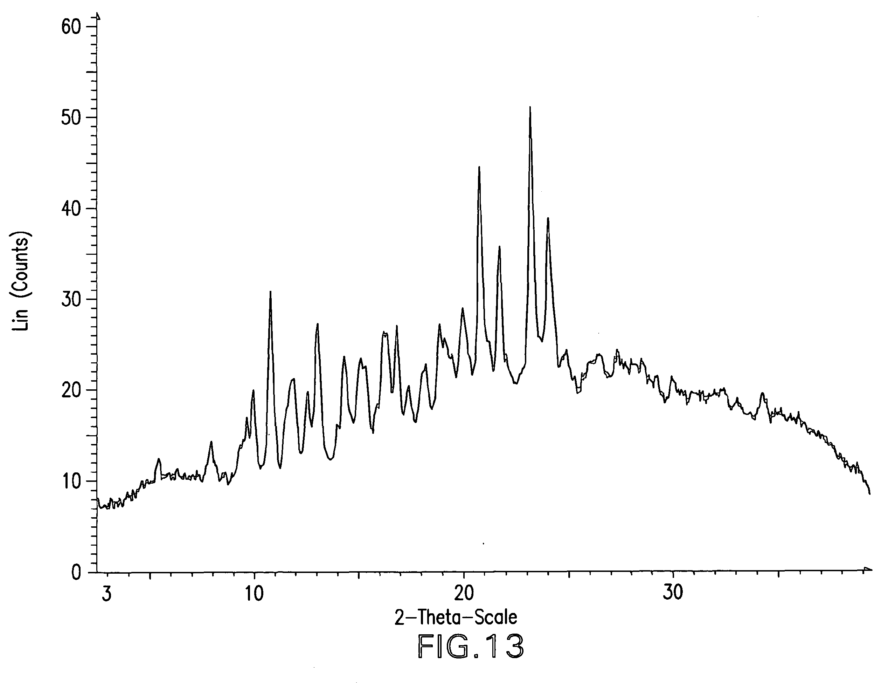

The present disclosure is further directed to the malonate salt of N-hydroxy-3-[4-[[[2-(2-methyl-1H-indol-3-yl)ethyl]amino]methyl]phenyl]-2E-2-propenamide, preferably the 2:1 malonate (hemi-malonate) salt of N-hydroxy-3-[4-[[[2-(2-methyl-1H-indol-3-yl)ethyl]amino]methyl]phenyl]-2E-2-propenamide. The malonate salt can be seen in the XRPD pattern shown in Figure 13. The hemi-malonate salt has an approximate aqueous solubility of 2 mg/mL. It is produced as a single, crystalline and anhydrous/unsolvated polymorph with a decomposition temperature above 170°C.

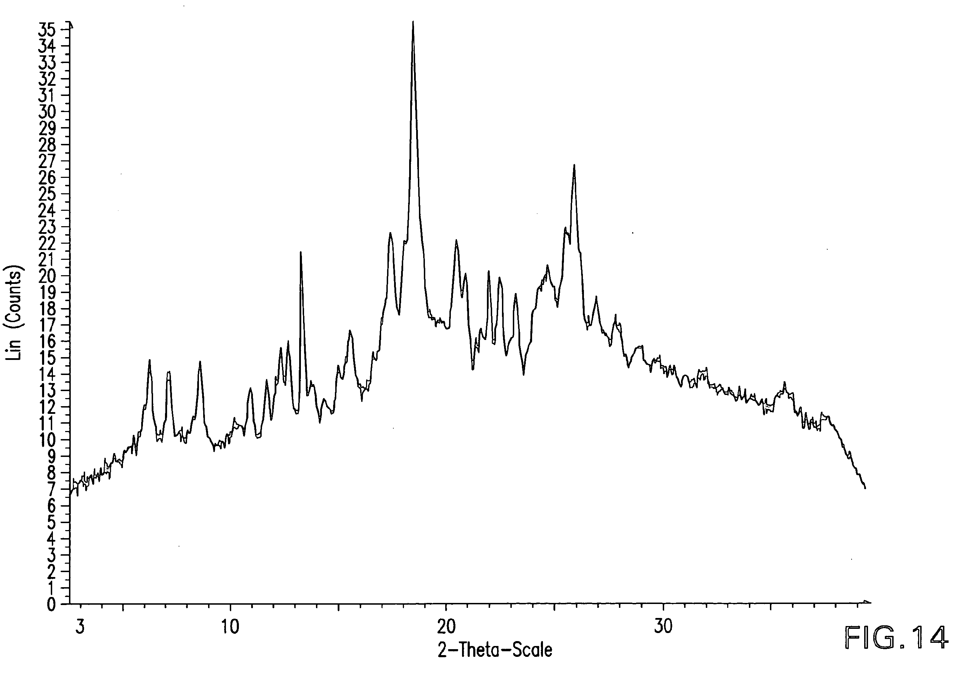

The present disclosure is further directed to the oxalate salt of N-hydroxy-3-[4-[[[2-(2-methyl-1H-indol-3-yl)ethyl]amino]methyl]phenyl]-2E-2-propenamide. The oxalate salt can be seen in the XRPD pattern shown in Figure 14.

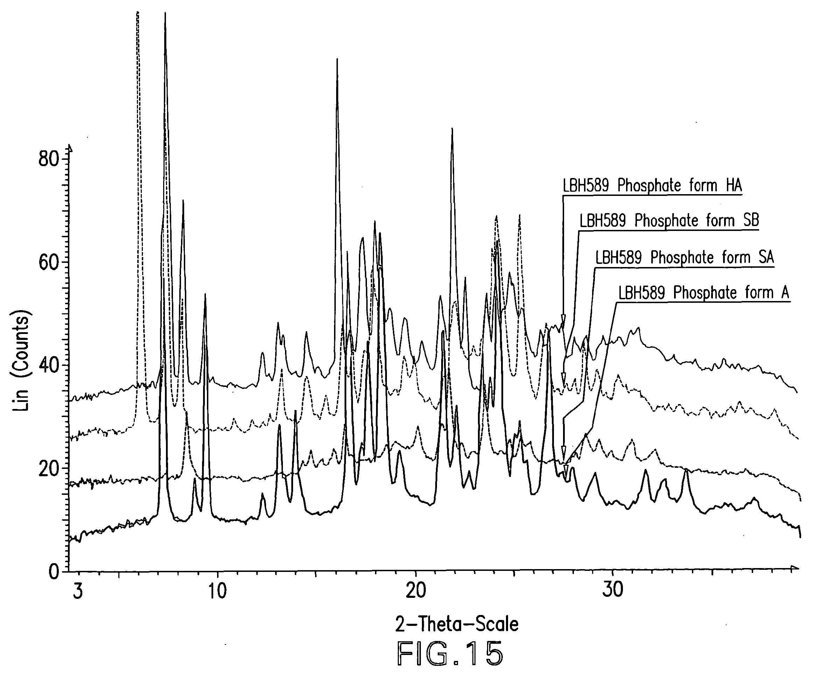

The present disclosure is further directed to the phosphate salt of N-hydroxy-3-[4-[[[2-(2-methyl-1H-indol-3-yl)ethyl]amino]methyl]phenyl]-2E-2-propenamide, preferably the 1:1 phosphate salt of N-hydroxy-3-[4-[[[2-(2-methyl-1H-indol-3-yl)ethyl]amino]methyl]phenyl]-2E-2-propenamide. Forms A, S_{A}, S_{B} and H_{A} for the phosphate salt can be seen in the XRPD patterns shown in Figure 15. Form A of the phosphate salt, isolated from reaction in acetone, has excellent crystallinity and a high decomposition temperature of 187°C. It exhibits a 1% LOD up to 165°C. It has an approximate aqueous solubility of 6 mg/mL. Form S_{A} of the phosphate salt, isolated from reaction in ethanol, has good crystallinity and exhibits a gradual weight loss on heating. It exhibits a 6.6% LOD up to 150°C. Form S_{B} of the phosphate salt, isolated from reaction in 2-propanol, has excellent crystallinity and exhibits a gradual weight loss on heating. It exhibits an around 7% LOD up to 150°C. Form H_{A} of the phosphate salt, a hydrate, isolated from reaction in ethanol and water (1:0.05), has excellent crystallinity and a high decomposition temperature of around 180°C. It exhibits a 7% LOD up to 150°C.

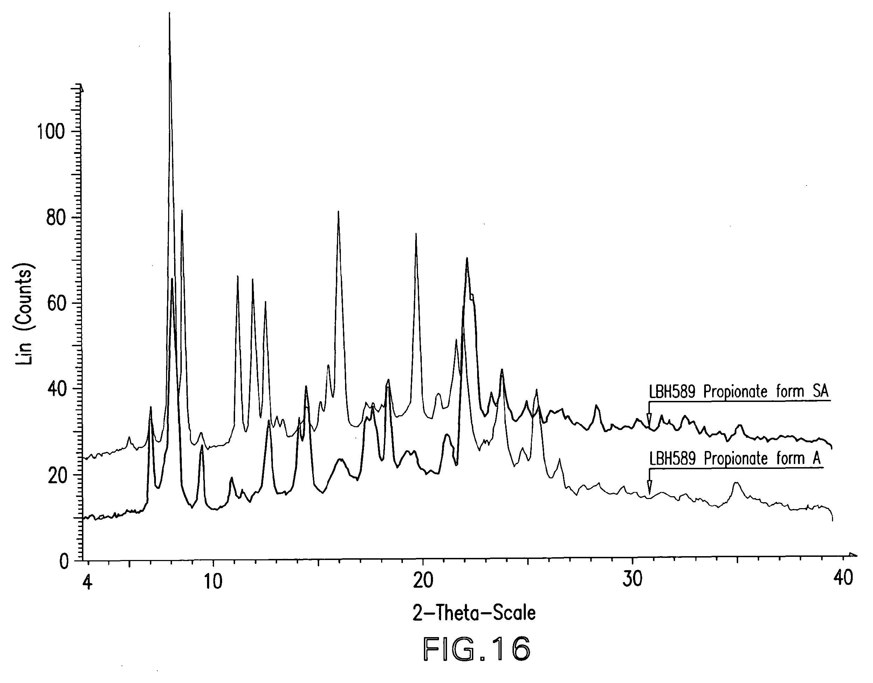

The present disclosure is further directed to the propionate salt of N-hydroxy-3-[4-[[[2-(2-methyl-1H-indol-3-yl)ethyl]amino]methyl]phenyl]-2E-2-propenamide, preferably the 1:1 propionate salt of N-hydroxy-3-[4-[[[2-(2-methyl-1H-indol-3-yl)ethyl]amino]methyl]phenyl]-2E-2-propenamide. Forms A and S_{A} for the propionate salt can be seen in the XRPD patterns shown in Figure 16. Form A of the propionate salt isolated from reaction in acetone has excellent crystallinity; its decomposition temperature is around 99°C. It exhibits an around 7% LOD up to 140°C. It has an approximate aqueous solubility of 4 mg/mL. Form S_{A} of the propionate salt, isolated from reaction in 2-propanol, is a 2-propanol solvate with excellent crystallinity. It exhibits a gradual weight loss on heating with an around 15% LOD up to 140°C.

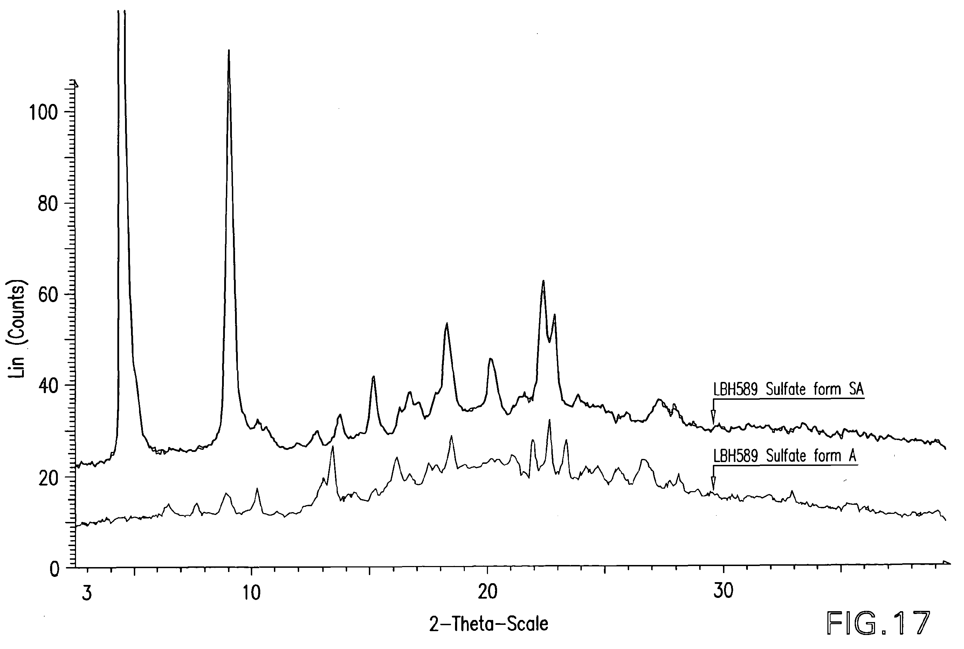

The present disclosure is further directed to the sulfate salt of N-hydroxy-3-[4-[[[2-(2-methyl-1H-indol-3-yl)ethyl]amino]methyl]phenyl]-2E-2-propenamide, preferably the 1:1 sulfate salt of N-hydroxy-3-[4-[[[2-(2-methyl-1H-indol-3-yl)ethyl]amino]methyl]phenyl]-2E-2-propenamide. Forms A and S_{A} for the sulfate salt can be seen in the XRPD patterns shown in Figure 17. Form A of the sulfate salt isolated from reaction in ethyl acetate as a yellow hygroscopic powder has poor crystallinity, a high decomposition temperature around 160°C, and exhibits an around 7% LOD up to 150°C. It is visibly hygroscopic at ambient conditions. Form S_{A} of the sulfate salt isolated from reaction in 2-propanol is a 2-propanol solvate with excellent crystallinity and a high decomposition temperature around 162°C. It exhibits an around 9-12% LOD up to 150°C.

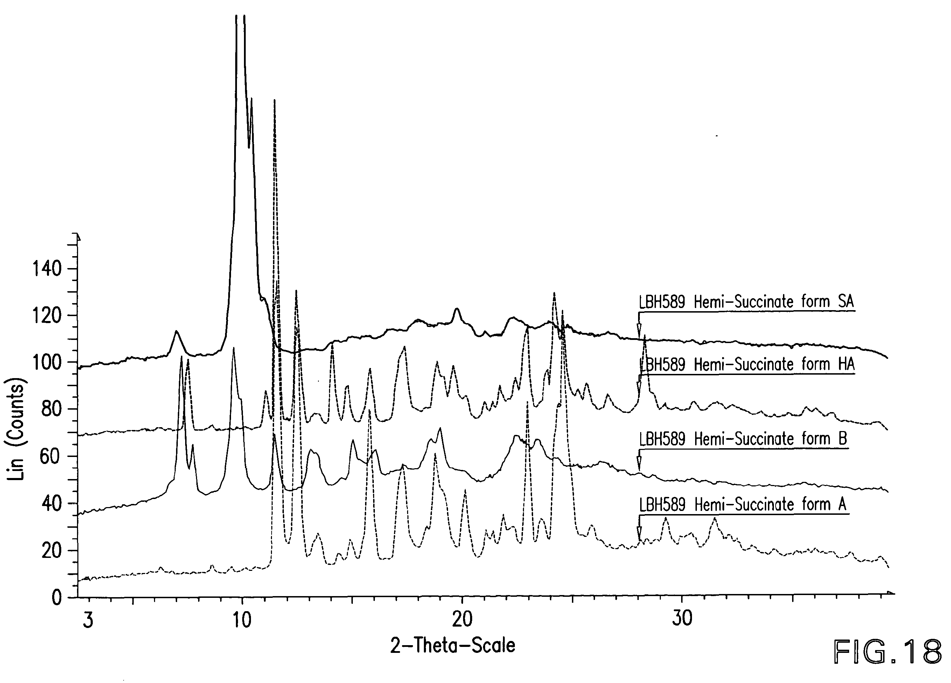

The present disclosure is further directed to the succinate salt of N-hydroxy-3-[4-[[[2-(2-methyl-1H-indol-3-yl)ethyl]amino]methyl]phenyl]-2E-2-propenamide, preferably the 2:1 succinate (hemi-succinate) salt of N-hydroxy-3-[4-[[[2-(2-methyl-1H-indol-3-yl)ethyl]amino]methyl]phenyl]-2E-2-propenamide. Forms A, B, H_{A} and S_{A} for the hemi-succinate salt can be seen in the XRPD patterns shown in Figure 18. Form A of the hemi-succinate salt reproducibly isolated from reaction in ethanol and water (1:0.05) or neat ethanol has excellent crystallinity and a very high decomposition temperature of around 204°C. It exhibits an around 1.1% LOD up to 200°C. It has an approximate aqueous solubility of 0.4 mg/mL. Form B of the hemi-succinate salt isolated from reaction in acetone or ethyl acetate has good crystallinity and a high decomposition temperature above 150°C. It exhibits a two-step LOD: around 1.5% up to 125°C and another 1.3-2.9% up to 150°C. Form S_{A} of the hemi-succinate salt isolated from reaction in 2-propanol is a 2-propanol solvate with good crystallinity and a high decomposition temperature around 155°C. It exhibits a two-step LOD: around 3% up to 70°C and another 4.6% up to 140°C. Form H_{A}, a monohydrate of the hemi-succinate salt, isolated from reaction in 2-propanol and water (1:0.05), has excellent crystallinity and a high decomposition temperature of around 180°C. It exhibits an around 4.6% LOD up to 160°C, corresponding to monohydrate.

The present disclosure is further directed to the sodium salt of N-hydroxy-3-[4-[[[2-(2-methyl-1H-indol-3-yl)ethyl]amino]methyl]phenyl]-2E-2-propenamide. This crystalline salt isolated as a yellow powder is visibly hygroscopic.

The present disclosure is further directed to the potassium salt of N-hydroxy-3-[4-[[[2-(2-methyl-1H-indol-3-yl)ethyl]amino]methyl]phenyl]-2E-2-propenamide. This crystalline salt isolated as a yellow powder is visibly hygroscopic.

The present disclosure is further directed to the calcium salt of N-hydroxy-3-[4-[[[2-(2-methyl-1H-indol-3-yl)ethyl]amino]methyl]phenyl]-2E-2-propenamide. This salt can be isolated as an amorphous material with an above-ambient glass transition temperature. Although amorphous, this salt was less hygroscopic than the sodium or potassium salts.

The present disclosure is further directed to the zinc salt of N-hydroxy-3-[4-[[[2-(2-methyl-1H-indol-3-yl)ethyl]amino]methyl]phenyl]-2E-2-propenamide. This salt can be isolated as an amorphous material with an above-ambient glass transition temperature. Although amorphous, this salt was less hygroscopic than the sodium or potassium salts.

The present invention also provides a pharmaceutical composition comprising:
(a) a therapeutically effective amount of a salt of N-hydroxy-3-[4-[[[2-(2-methyl-1H-indol-3-yl)ethyl]amino]methyl]phenyl]-2E-2-propenamide, as described in the claims; and
(b) at least one pharmaceutically acceptable carrier, diluent, vehicle or excipient.

A "therapeutically effective amount" is intended to mean the amount of the inventive salt that, when administered to a subject in need thereof, is sufficient to effect treatment for disease conditions alleviated by the inhibition of histone deacetylase activity. The amount of a given compound of the invention that will be therapeutically effective will vary depending upon factors such as the disease condition and the severity thereof, the identity of the subject in need thereof, etc., which amount may be routinely determined by artisans of ordinary skill in the art.

The at least one pharmaceutically acceptable carrier, diluent, vehicle or excipient can readily be selected by one of ordinary skill in the art and will be determined by the desired mode of administration. Illustrative examples of suitable modes of administration include oral, nasal, parenteral, topical, transdermal and rectal. The pharmaceutical compositions of this invention may take any pharmaceutical form recognizable to the skilled artisan as being suitable. Suitable pharmaceutical forms include solid, semisolid, liquid or lyophilized formulations, such as tablets, powders, capsules, suppositories, suspensions, liposomes and aerosols.

There is also described a compound for use in a method of treating a disease which responds to an inhibition of histone deacetylase activity comprising the step of administering to a subject in need of such treatment a therapeutically effective amount of a salt of N-hydroxy-3-[4-[[[2-(2-methyl-1H-indol-3-yl)ethyl]amino]methyl]phenyl]-2E-2-propenamide. As noted above, illustrative modes of administration include oral, nasal, parenteral, topical, transdermal and rectal. Administration of the crystalline form may be accomplished by administration of a pharmaceutical composition of the ninth embodiment of the invention or via any other effective means.

Specific embodiments of the invention will now be demonstrated by reference to the following examples.

In the following examples, with regard to crystallinity, "excellent" refers to a material having XRPD main peaks which are sharp and have intensities above 70 counts; "good" refers to a material having XRPD main peaks which are sharp and have intensities within 30-70 counts; and "poor" refers to a material having XRPD main peaks which are broad and have intensities below 30 counts. In addition, "LOD" refers to weight loss determined between ambient and decomposition temperatures. The later is approximated by the onset of the first derivative of the thermogravimetric curve vs. temperature. This is not the true onset, since weight loss does not occur with the same rate for all the salts. Hence, the actual decomposition temperature may be lower than that stated. Salt formation, stoichiometry and the presence or absence of solvents is confirmed by observing the ¹H-NMR chemical shifts of the corresponding salt forming agents and reaction solvents (the tables contain one characteristic chemical shift for salt forming agents or solvents). Water content could not be extracted from the NMR data, because the water peaks were broad. The extent of protonation of the free base is assessed by the change in the chemical shift of the benzylic (H_{bz}) protons. Further, salts of the present invention precipitated out as free-flowing powders (FFP), sticky amorphous materials (SAM) (which had a gummy consistency that tended to agglomerate, forming a single spherical mass or stick to the walls of the reaction vessel) or amorphous gels (AG). Finally, "-" indicates a measurement not taken.

### Reference EXAMPLE 1

### Preparation of Acetate Salt

About 40-50 mg of N-hydroxy-3-[4-[[[2-(2-methyl-1H-indol-3-yl)ethyl]amino]methyl]phenyl]-2E-2-propenamide free base monohydrate was suspended in 1 mL of a solvent as listed in Table 1. A stoichiometric amount of acetic acid was subsequently added to the suspension. The mixture was stirred at either 60°C or ambient temperature (where a clear solution formed, stirring continued at 4°C). Solids were collected by filtration and analyzed by XRPD, TGA and in some instances ¹H-NMR.

**Table 1**

| **Solvent** | **T, °C** | **Physical Appearance** | **Crystallinity and Form** | **LOD, % T_{decomposition} (T_{desolvation})** | **¹H-NMR** |
|---|---|---|---|---|---|
| Acetone | Ambient | FFP | Excellent S_{A} | 13.5 (107.9) | 1.89 (acetate, 3H) |
| | | | | 147.9 | 2.08 (acetone, 6H) |
| | | | | | 3.74 (H_{bz}) |
| IPA | 60 | FFP | Good A | -10.5 (72.5) | - |
| | | | | 148.7 | |
| AcOEt | 60 | FFP | Good A | 9.3 (105.1) | 1.89 (acetate, 3H) |
| | | | | 147.9 | 3.73 (H_{bz}) |

The salt forming reaction in acetone produced a highly crystalline salt, with the ratio of N-hydroxy-3-[4-[[[2-(2-methyl-1H-indol-3-yl)ethyl]amino]methyl]phenyl]-2E-2-propenamide to acetate of 1:1, identified as a stoiciometric acetone solvate S_{A}. The salt forming reaction in isopropyl alcohol and ethyl acetate at 60°C produced the same crystalline, non-solvated acetate salt (form A). The accompanied weight loss above 105°C is either due to the loss of water (if the salt is a hydrate) or loss of acetic acid or both.

### Reference EXAMPLE 2

### Preparation of Benzoate Salt

About 40-50 mg of N-hydroxy-3-[4-[[[2-(2-methyl-1H-indol-3-yl)ethyl]amino]methyl]phenyl]-2E-2-propenamide free base monohydrate was suspended in 1 mL of a solvent as listed in Table 2. A stoichiometric amount of benzoic acid was subsequently added to the suspension. The mixture was stirred at ambient temperature (where a clear solution formed, stirring continued at 4°C). Solids were collected by filtration and analyzed by XRPD, TGA and in some instances ¹H-NMR.

**Table 2**

| **Solvent** | **T, °C** | **Physical Appearance** | **Crystallinity and Form** | **LOD % T_{decomposition}** | **¹H-NMR** |
|---|---|---|---|---|---|
| EtOH:H₂O (1:0.05) | Ambient | FFP | Excellent S_{A} | 1.5 | - |
| | | | | (prior to dec. at 110°C) | |
| IPA:H₂O (1:0.05) | Ambient | FFP | Excellent S_{B} | 6.3 * | 1.02 (IPA, 6H) |
| | | | | (isothermal at 120°C) | 3.83 (H_{bz}) |
| EtOH | Ambient | FFP | Excellent S_{A} | 5.2 * | 1.04 (EtOH, 5H) |
| | | | | (isothermal at 120°C) | 3.43 (EtOH, 1H) |
| | | | | | 7.93 (benzoate, 2H) |
| | | | | | 3.85 (H_{bz}) |
| IPA | Ambient | FFP | Excellent S_{B} | 1.5% | - |
| | | | | (prior dec. at 100°C) | |
| Acetone | Ambient | FFP | Excellent A | 0.5% | 7.93 (benzoate, 2H) |
| | | | | 160.2 | 3.84 (H_{bz}) |

| | | | | | |
|---|---|---|---|---|---|
| *Isothermal hold at 120°C for 10 minutes | | | | | |

The salt forming reaction in ethanol alone and with water produced the same ethanol solvate S_{A}. The stoichiometry of the protonated base:benzoate:ethanol is 1:1:0.5 by NMR. Solvent loss and decomposition are closely spaced events at the heating rate of 10°C/min., and the ethanol content could not be determined initially. Eventually, it was determined by holding at 120°C for 10 min. The LOD of 5.2 % corresponds to 0.5 moles of ethanol per formula unit. Isopropyl alcohol alone and with water produced the same isopropanol (IPA) solvate SB. The stoichiometry of the protonated base:benzoate is 1:1 by NMR. Solvent loss and decomposition are closely spaced at the heating rate of 10°C/min., and the isopropanol content could not be determined initially. Eventually, it was determined by holding at 120°C for 10 min. The 6.3% LOD corresponds to 0.5 moles of IPA per formula unit. Based on solvent content and XRPD patterns, the two solvates SA and SB appeared to be isostructural. The salt forming reaction in acetone produced benzoate salt that did not contain any solvent or water, a 1:1 stoichiometric salt of excellent crystallinity and high decomposition temperature (form A).

### Reference EXAMPLE 3

### Formation of Hydrochloride Salt

About 40-50 mg of N-hydroxy-3-[4-[[[2-(2-methyl-1H-indol-3-yl)ethyl]amino]methyl]phenyl]-2E-2-propenamide free base was suspended in 1 mL of a solvent as listed in Table 3. A stoichiometric amount of hydrochloric acid was subsequently added to the suspension. The mixture was stirred at either 60°C or ambient temperature (where a clear solution formed, stirring continued at 4°C). Solids were collected by filtration and analyzed by XRPD, TGA and in some instances ¹H-NMR.

**Table 3**

| **Solvent** | **T, °C** | **Physical Appearance** | **Crystallinity and Form** | **LOD,** % **T_{decomposit.}** | **¹H-NMR** |
|---|---|---|---|---|---|
| EtOH:H₂O (1:0.05) | 60 | Clear solution to FFP | Excellent A | 0.5 | 4.20 (H_{bz}) |
| EtOH | Ambient | Clear solution to FFP | Excellent A | 1.1 232.3 | 4.19 (H_{bz}) |
| IPA | Ambient | FFP yellow to white powder | Excellent A | - | 4.18 (H_{bz}) |
| Acetone | Ambient | FFP to SAM to FFP | Excellent A | - | 4.18 (H_{bz}) |
| AcOEt | Ambient | FFP to SAM to FFP | Excellent A | - | - |

All the above five reactions produced the same crystalline salt. The salt was anhydrous and decomposed at high temperature.

### Reference EXAMPLE 4

### Formation of Hemi-Citrate Salt

About 40-50 mg of N-hydroxy-3-[4-[[[2-(2-methyl-1H-indol-3-yl)ethyl]amino]methyl]phenyl]-2E-2-propenamide free base was suspended in 1 mL of a solvent as listed in Table 4. A stoichiometric amount of citric acid was subsequently added to the suspension. The mixture was stirred at either 60°C or ambient temperature (where a clear solution formed, stirring continued at 4°C). Solids were collected by filtration and analyzed by XRPD, TGA and in some instances ¹H-NMR.

**Table 4**

| **Solvent** | **T, °C** | **Physical Appearance** | **Crystallinity and Form** | **LOD, % T_{decomposit.}** | **¹H-NMR** |
|---|---|---|---|---|---|
| IPA:H₂O (1:0.05) | 60 | SAM to FFP | Excellent A | 0.4 184.3 | 3.98 (H_{bz}) |
| Acetone | Ambient 60 | FFP to SAM to FFP | Excellent A | 5.0 to 5.8 | - |
| EtOH | 60 | SAM to FFP | Excellent A | - | - |
| IPA:H₂O (1:0.025) | 60 | SAM to FFP | Excellent A | 0.3 | - |
| | | | | 181.0 | |
| IPA:H₂O (1:0.05) | 60 | SAM to FFP | Excellent A | - | - |
| Acetone:H₂O (1:0.025) | 60 | SAM to FFP | Excellent A | - | - |
| Acetone:H₂O (1:0.05) | 60 | SAM to FFP | Excellent A | 0.7 | - |
| | | | | 177.0 | |

Heating to 60°C (acetone and ethanol), as well as the introduction of water (isopropyl alcohol and water, acetone and water at 60°C) yielded a highly crystalline salt that does not contain any solvent or water. A high LOD value for acetone at ambient/60°C is due to the presence of amorphous material within the crystalline powder. The stoichiometry of the salt could not be determined by ¹H-NMR in DMSO-d₆, since the expected peak for the citrate ion coincides with that of the solvent. However, ¹³C-NMR spectroscopy indicated the presence of two ¹³C = O signals at 177.3 and 171.6 ppm. The former is due to the protonated carboxylic group and the latter to the unprotonated carboxylate.

### Reference EXAMPLE 5

### Formation of Hemi-Fumarate Salt

About 40-50 mg of N-hydroxy-3-[4-[[[2-(2-methyl-1H-indol-3-yl)ethyl]amino]methyl]phenyl]-2E-2-propenamide free base monohydrate was suspended in 1 mL of a solvent as listed in Table 5. A stoichiometric amount of fumaric acid was subsequently added to the suspension. The mixture was stirred at either 60°C or ambient temperature (where a clear solution formed, stirring continued at 4°C). Solids were collected by filtration and analyzed by XRPD, TGA and in some instances ¹H-NMR.

**Table 5**

| **Solvent** | **T, °C** | **Physical Appearance** | **Crystallinity and Form** | **LOD, % T_{decomposit.}** | **¹H-NMR** |
|---|---|---|---|---|---|
| EtOH | Ambient | FFP to SAM to FFP | Excellent B | 1.1 + 1.7 | 3.93 (H_{bz}) |
| | | | | (2-step) 213.2 | 6.50 (1H, fumarate) |
| IPA | Ambient | FFP | Consists of one intense peak H_{A} | 3.4 + 6.0 | 3.91 (H_{bz}) |
| | | | | (2-step) 159.8 | 6.50 (1H, fumarate) |
| | | | | | only small amount of IPA |
| EtOH:H₂O (1:0.05) | Ambient | FFP to SAM to FFP | Excellent A | 0.7 | 3.90 (H_{bz}) |
| | | | | 217.4 | 6.49 (1H, fumarate) |
| IPA:H₂O (1:0.05) | Ambient | FFP | Excellent A | 1.5 | - |
| | | | | 208.2 | |
| IPA:H₂O (1:0.05) | Ambient | FFP | Excellent A | - | - |
| EtOH:H₂O (1:0.025) | Ambient | FFP to SAM to FFP | Poor A | 0.7 | - |
| | | | | 154.8 | |
| EtOH:H₂O (1:0.05) | Ambient | FFP to SAM to FFP | Excellent A | 0.9 | 3.90 (H_{bz}) |
| | | | | 217.1 | 6.49 (1H, fumarate) |

The salt forming reactions in isopropyl alcohol and acetone at ambient temperature produced fumarate salts of stoichiometry 2:1 (protonated base:fumarate), i.e., hemi-fumarate salts. Although none of them was a solvate, they had poor crystallinity and a low decomposition temperature. The LOD for isopropyl alcohol at ambient temperature was most likely associated with the loss of water. The salt forming reaction in ethanol, ethanol and water, and isopropyl alcohol and water, all at ambient temperature or 60°C, produced a fumarate salt of stoichiometry 2:1 (protonated base:fumarate), i.e., hemi-fumarate salt. The salt forming reaction in ethanol and water and isopropyl alcohol and water (1:0.05), ambient or 60°C, produced identical XRPD spectra (anhydrous form A). The spectrum of the salt formed by ethanol at ambient temperature, albeit similar, displays some small differences and it may represent a unique, hemi-fumarate polymorph (form B) of similar structure.

### Reference EXAMPLE 6

### Formation of Gentisate Salt

About 40-50 mg of N-hydroxy-3-[4-[[[2-(2-methyl-1H-indol-3- yl)ethyl]amino]methyl]phenyl]-2E-2-propenamide free base was suspended in 1 mL of a solvent as listed in Table 6. A stoichiometric amount of 2,5-dihydroxybenzoic acid (gentisic acid) was subsequently added to the suspension. The mixture was stirred at either 60°C or ambient temperature (where a clear solution formed, stirring continued at 4°C). Solids were collected by filtration and analyzed by XRPD, TGA and in some instances ¹H-NMR.

**Table 6**

| **Solvent** | **T, °C** | **Physical Appearance** | **Crystallinity and Form** | **LOD, % T_{decomposit.}** | **¹H-NMR** |
|---|---|---|---|---|---|
| EtOH:H₂O (1:0.05) | 60 | Clear solution to FFP | Excellent A | 0.3 | 4.18 (H_{bz}) |
| | | | | 235.5 | 6.61 (1H, gentisate) |

The gentisate salt prepared was highly crystalline, anhydrous, and decomposed at a very high temperature. The stoichiometry of the salt is 1:1 by NMR.

### Reference EXAMPLE 7

### Formation of Monohydrate DL-lactate Salt

About 40-50 mg of N-hydroxy-3-[4-[[[2-(2-methyl-1H-indol-3-yl)ethyl]amino]methyl]phenyl]-2E-2-propenamide free base was suspended in 1 mL of a solvent as listed in Table 7. A stoichiometric amount of lactic acid was subsequently added to the suspension. The mixture was stirred at ambient temperature and when a clear solution formed, stirring continued at 4°C. Solids were collected by filtration and analyzed by XRPD, TGA and ¹H-NMR.

**Table 7**

| **Solvent** | **T, °C** | **Physical Appearance** | **Crystallinity and Form** | **LOD, % T_{decomposit.}** | **¹H-NMR** |
|---|---|---|---|---|---|
| IPA | 4 | FFP | Excellent H_{A} | 4.3 (79.3) | - |
| | | | | 156.3 | |
| Acetone | 4 | FFP | Excellent H_{A} | 4.5 (77.8) | 4.18 (H_{bz}) |
| | | | | 149.5 | |

The salt forming reaction in isopropyl alcohol and acetone at 4°C produced a stoichiometric (1:1) DL-lactate salt, a monohydrate. The salt is crystalline, begins to dehydrate above 77°C, and decomposes above 150°C.

### Reference EXAMPLE 8

### Formation of Maleate Salt

About 40-50 mg of N-hydroxy-3-[4-[[[2-(2-methyl-1H-indol-3-yl)ethyl]amino]methyl]phenyl]-2E-2-propenamide free base monohydrate was suspended in 1 mL of a solvent as listed in Table 8. A stoichiometric amount of maleic acid was subsequently added to the suspension. The mixture was stirred at either 60°C or ambient temperature (where a clear solution formed, stirring continued at 4°C). Solids were collected by filtration and analyzed by XRPD, TGA and in some instances ¹H-NMR.

**Table 8**

| **Solvent** | **T, °C** | **Physical Appearance** | **Crystallinity and Form** | **LOD, % T_{decomposit.}** | **¹H-NMR** |
|---|---|---|---|---|---|
| EtOH | RT to 4 | Clear solution to FFP | Excellent H_{A} ? | 6.2 (RT) | 4.22 (H_{bz}) |
| | | | | 150 | 6.01 (2H, maleate) |
| IPA | 60 | SAM to FFP | Excellent A | 0.2 | 4.22 (H_{bz}) |
| | | | | 178.1 | 6.01 (2H, maleate) |
| Acetone | 60 | SAM to FFP | Excellent A | 0.2 | 4.22 (H_{bz}) |
| | | | | 176.1 | 6.01 (2H, maleate) |

The salt forming reaction in isopropyl alcohol and acetone at 60°C produced highly crystalline, anhydrous solids that decompose above ∼180°C. Maleic acid was the only dicarboxylic acid that produced a 1:1 salt with N-hydroxy-3-[4-[[[2-(2-methyl-1H-indol-3-yl)ethyl]amino]methyl]phenyl]-2E-2-propenamide. Its ¹H-NMR spectrum displays a resonance at 6.01 ppm, corresponding to the two olefinic protons, and a resonance at 10.79 ppm due to one unprotonated carboxylic acid. Maleic acid also formed a salt with high water content that is lost under mild heating conditions. It is likely that the salt forming reaction in ethanol (RT to 4°C) produced a hydrate (form H_{A}).

### Reference EXAMPLE 9

### Formation of Hemi-Malate Salt

About 40-50 mg of N-hydroxy-3-[4-[[[2-(2-methyl-1H-indol-3-yl)ethyl]amino]methyl]phenyl]-2E-2-propenamide free base monohydrate was suspended in 1 mL of a solvent as listed in Table 9. A stoichiometric amount of malic acid was subsequently added to the suspension. The mixture was stirred at either 60°C or ambient temperature (where a clear solution formed, stirring continued at 4°C). Solids were collected by filtration and analyzed by XRPD, TGA and in some instances ¹H-NMR.

**Table 9**

| **Solvent** | **T, °C** | **Physical Appearance** | **Crystallinity and Form** | **LOD, % T_{decomposit.}** | **¹H-NMR** |
|---|---|---|---|---|---|
| EtOH:H₂O | 60 | SAM to FFP | Excellent A | 1.9 | 3.96 (H_{bz}) |
| (1:0.05) | | | | 206.0 | 3.83 (0.5H, malate) |
| EtOH | 60 | SAM to FFP | Excellent A | 0.4 | - |
| | | | | 199.3 | |
| IPA | 60 | SAM to FFP | Excellent A | - | - |
| Acetone | 60 | SAM to FFP | Excellent S_{A} | 0.6 | 3.97 (H_{bz}) |
| | | | | 95 | 3.84 (0.5H, malate) |
| EtOH:H₂O (1:0.05) | Ambient | SAM to FFP | Excellent A | - | - |

The salt forming reaction in ethanol and water, ethanol and isopropyl alcohol produced the same crystalline and anhydrous hemi-malate salt. The difference in LOD between ethanol and water (1:0.05) and ethanol may reflect varying amounts of amorphous material in the two samples. The salt forming reaction in acetone afforded a different hemi-malate salt that continuously loses weight above ∼95°C. This salt is an acetone solvate (form S_{A}). Solvent loss and decomposition are closely spaced thermal events.

### Reference EXAMPLE 10

### Formation of Hemi-Malonate Salt

About 40-50 mg of N-hydroxy-3-[4-[[[2-(2-methyl-1H-indol-3-yl)ethyl]amino]methyl]phenyl]-2E-2-propenamide free base was suspended in 1 mL of a solvent as listed in Table 10. A stoichiometric amount malonic acid was subsequently added to the suspension. The mixture was stirred at either 60°C or ambient temperature (where a clear solution formed, stirring continued at 4°C). Solids were collected by filtration and analyzed by XRPD, TGA and in some instances ¹H-NMR.

**Table 10**

| **Solvent** | **T, °C** | **Physical Appearance** | **Crystallinity and Form** | **LOD, % T_{decomposit.}** | **¹H-NMR** |
|---|---|---|---|---|---|
| EtOH | 60 | SAM to FFP | Poor A | 1.0 | |
| | | | | 169.5 | |
| IPA | 60 | SAM to FFP | Good A | 1.5 | 4.00 (H_{bz}) |
| | | | | 174.1 | 2.69 (1H, malonate) |
| Acetone | 60 | SAM to FFP | Good A | - | - |
| Acetone | Ambient | FFP to SAM to FFP | Good A | - | - |

All reactions afforded the same hemi-malonate salt. The crystallinity is usually good, although an amorphous halo could be seen in all the XRPD spectra. The water associated with these materials is likely due to increased moisture sorption by the amorphous component. Ambient conditions during synthesis appear to produce a better quality salt.

### Reference EXAMPLE 11

### Formation of Mesylate Salt

About 40-50 mg of N-hydroxy-3-[4-[[[2-(2-methyl-1H-indol-3-yl)ethyl]amino]methyl]phenyl]-2E-2-propenamide free base monohydrate was suspended in 1 mL of a solvent as listed in Table 11. A stoichiometric amount of methanesulfonic acid was subsequently added to the suspension. The mixture was stirred at either 60°C or ambient temperature (where a clear solution formed, stirring continued at 4°C). Solids were collected by filtration and analyzed by XRPD, TGA and in some instances ¹H-NMR.

**Table 11**

| **Solvent** | **T, °C** | **Physical Appearance** | **Crystallinity and Form** | **LOD, % T_{decomposit.}** | **¹H-NMR** |
|---|---|---|---|---|---|
| Acetone | 60 | SAM to FFP | Excellent A + B ? | 1.6 | 4.22 (H_{bz}) |
| + | | | | 172.8 | 2.33 (∼5H, methane sulfonate) |
| AcOEt | Ambient | FFP | Excellent A | 1.3 + 1.3 (2-step) | 4.22 (H_{bz}) |
| | | | | 170.9 | 2.36 (∼5H, methane sulfonate) |

The salt forming reaction in ethyl acetate afforded a yellow salt, upon stirring at room temperature. The salt (form A) is crystalline, displays a 2-step weight loss and, by NMR, does not contain any solvent but appears to have more than one molecule of methanesulfonate (mesylate). The salt forming reaction in acetone afforded isolation of a white powder after heating at 60°C. It displayed excellent crystallinity but may be a composite of more than one polymorphic form (forms A and B). By NMR, it does not contain any solvent but appears to contain more than one molecule of methanesulfonate. Another salt forming reaction in ethyl acetate, in which reaction is initiated at ambient temperature and then the obtained yellowish powder suspension is heated to 50°C, afforded isolation of a new form B, as shown in Figure 5.

### Reference EXAMPLE 12

### Formation of Oxalate Salt

About 40-50 mg of N-hydroxy-3-[4-[[[2-(2-methyl-1H-indol-3-yl)ethyl]amino]methyl]phenyl]-2E-2-propenamide free base was suspended in 1 mL of a solvent as listed in Table 12. A stoichiometric amount of salt forming agent oxalic acid was subsequently added to the suspension. The mixture was stirred at either 60°C or ambient temperature (where a clear solution formed, stirring continued at 4°C). Solids were collected by filtration and analyzed by XRPD, TGA and in some instances ¹H-NMR.

**Table 12**

| **Solvent** | **T, °C** | **Physical Appearance** | **Crystallinity and Form** | **LOD, % T_{decomposit.}** | **¹H-NMR** |
|---|---|---|---|---|---|
| EtOH:H₂O (1:0.05) | 60 | FFP | Poor | - | - |
| IPA:H₂O (1:0.05) | 60 | FFP | Poor | - | - |
| EtOH | Ambient | Waxy solid | Amorphous | - | - |
| IPA | Ambient | Waxy solid | Amorphous | - | - |
| Acetone | Ambient | Waxy solid | Amorphous | - | - |

Oxalate salts, although precipitated immediately upon addition of oxalic acid to suspensions of N-hydroxy-3-[4-[[[2-(2-methyl-1H-indol-3-yl)ethyl]amino]methyl]phenyl]-2E-2-propenamide, were hard to isolate and appear to absorb water during filtration.

### Reference EXAMPLE 13

### Formation of Phosphate Salt

About 40-50 mg of N-hydroxy-3-[4-[[[2-(2-methyl-1H-indol-3-yl)ethyl]amino]methyl]phenyl]-2E-2-propenamide free base monohydrate was suspended in 1 mL of a solvent as listed in Table 13. A stoichiometric amount of phosphoric acid was subsequently added to the suspension. The mixture was stirred at either 60°C or ambient temperature (where a clear solution formed, stirring continued at 4°C). Solids were collected by filtration and analyzed by XRPD, TGA and in some instances ¹H-NMR.

**Table 13**

| **Solvent** | **T, °C** | **Physical Appearance** | **Crystallinity and Form** | **LOD, % T_{decomposit.}** | **¹H-NMR** |
|---|---|---|---|---|---|
| EtOH:H₂O (1:0.05) | 60 | FFP | Excellent H_{A} | 7.0 | 3.94 (H_{bz}) |
| | | | | 179.6 | |
| EtOH | Ambient | FFP | Good S_{A} | ∼ 6.6 | 1.1 (∼1.5 H, EtOH) |
| | | | | | 4.00 (H_{bz}) |
| IPA | Ambient | FFP | Excellent S_{B} | ∼ 7.0 | 1.02 (3-4 H, IPA) |
| | | | | | 4.00 (H_{bz}) |
| Acetone | RT to 60 | SAM to FFP | Excellent A | 1.0 | 4.00 (H_{bz}) |
| | | | | 187.4 | |
| AcOEt | RT to 60 | SAM to FFP | Good A | 1.2 | - |
| | | | | 175.5 | |

The salt forming reaction in ethanol and isopropyl alcohol gave ethanol and isopropanol hemi-solvates (forms S_{A} and S_{B}, respectively). In ethanol and water, only traces of ethanol were detected by NMR, in spite of the large LOD. The material is either hygroscopic or a hydrate (form H_{A}) that loses water upon gentle heating and vacuum conditions (the loss of water measured by TGA is complete in by ∼60°C at 10°C/min.). The salt forming reaction in acetone and ethyl acetate produced the same crystalline and anhydrous phosphate salt (form A). The stoichiometry is most likely 1:1. The salt displays a high decomposition temperature.

### Reference EXAMPLE 14

### Formation of Propionate Salt

About 40-50 mg of N-hydroxy-3-[4-[[[2-(2-methyl-1H-indol-3-yl)ethyl]amino]methyl]phenyl]-2E-2-propenamide free base monohydrate was suspended in 1 mL of a solvent as listed in Table 14. A stoichiometric amount of propionic acid was subsequently added to the suspension. The mixture was stirred at either 60°C or ambient temperature (where a clear solution formed, stirring continued at 4°C). Solids were collected by filtration and analyzed by XRPD, TGA and in some instances ¹H-NMR.

**Table 14**

| **Solvent** | **T, °C** | **Physical Appearance** | **Crystallinity and Form** | **LOD, % T_{decomposit.}** | **¹H-NMR** |
|---|---|---|---|---|---|
| IPA | 60 | FFP | Excellent S_{A} | 15.1 | 0.97 (3H, propionic) |
| | | | | | 1.02 (∼4H, IPA) |
| | | | | | 3.73 (H_{bz}) |
| Acetone | 60 | FFP | Excellent A | 7.0 | 0.97 (3H, propionic) |
| | | | | 98.9 | 3.73 (Hbz) |
| AcOEt | 60 | FFP | Excellent A | 6.3 | - |
| | | | | -100 | |

A salt forming reaction in ethanol afforded the unreacted free base (most likely form H_{B}). Isopropyl alcohol produced an IPA solvate of the propionate salt (form S_{A}). Based on NMR, the IPA content is -0.5. The salt shows a weight loss of 15%, which corresponds to the loss of IPA plus an unidentified component. The salt forming reaction in acetone and ethyl acetate produced the same crystalline and unsolvated salt (form A). A weight loss of 6.3-7%, that starts at ∼100°C, is due to water (if the salt is a hydrate), propionic acid or a decomposition product. Upon completion of weight loss (∼140°C), the salt decomposes. It should be pointed out that when the material is dissolved in DMSO for NMR, free propionic acid and only traces of propionate were detected.

### Reference EXAMPLE 15

### Formation of Sulfate Salt

About 40-50 mg of N-hydroxy-3-[4-[[[2-(2-methyl-1H-indol-3-yl)ethyl]amino]methyl]phenyl]-2E-2-propenamide free base monohydrate was suspended in 1 mL of a solvent as listed in Table 15. A stoichiometric amount of sulfuric acid was subsequently added to the suspension. The mixture was stirred at either 60°C or ambient temperature (where a clear solution formed, stirring continued at 4°C). Solids were collected by filtration and analyzed by XRPD, TGA and in some instances ¹H-NMR.

**Table 15**

| **Solvent** | **T, °C** | **Physical Appearance** | **Crystallinity and Form** | **LOD, % T_{decomposit.}** | **¹H-NMR** |
|---|---|---|---|---|---|
| IPA | 60 | SAM to FFP | Excellent S_{A} | 8.9 to 12 | 1.02 (6H, IPA) |
| | | | | 162 | 1.10 (3H, IPA⁺) |
| | | | | | 4.22 (H_{bz}) |
| AcOEt | Ambient | FFP | Poor A | ∼ 6.7 | 4.22 (H_{bz}) |
| | | | | ∼ 160 | |

The salt forming reaction in isopropyl alcohol afforded isolation of a white crystalline salt. It was identified as an isopropanol solvate (form S_{A}), containing 1.5 mol of IPA per formula unit. In DMSO, 0.5 mol of IPA is protonated. The salt forming reaction in ethyl acetate afforded isolation of a yellow hygroscopic powder (form A). During filtration, the sample visibly absorbed moisture, and its poor crystallinity is attributed to this effect.

### Reference EXAMPLE 16

### Formation of Hemi-Succinate Salt

About 40-50 mg of N-hydroxy-3-[4-[[[2-(2-methyl-1H-indol-3-yl)ethyl]amino]methyl]phenyl]-2E-2-propenamide free base monohydrate was suspended in 1 mL of a solvent as listed in Table 16. A stoichiometric amount of succinic acid was subsequently added to the suspension. The mixture was stirred at either 60°C or ambient temperature (where a clear solution formed, stirring continued at 4°C). Solids were collected by filtration and analyzed by XRPD, TGA and in some instances ¹H-NMR.

**Table 16**

| **Solvent** | **T, °C** | **Physical Appearance** | **Crystallinity and Form** | **LOD, % T_{decomposit.}** | **¹H-NMR** |
|---|---|---|---|---|---|
| EtOH:H₂O (1:0.05) | 60 | SAM to FFP | Excellent A | 1.1 | 2.31 (2H, succinate) |
| | | | | 203.7 | 3.86 (H_{bz}) |
| IPA:H₂O (1:0.05) | 60 | SAM to FFP | Excellent H_{A} | 4.6 | 2.31 (2H, succinate) |
| | | | | | 3.85 (H_{bz}) |
| EtOH | Ambient | FFP to SAM to FFP | Excellent A | 1.1 | 2.31 (2H, succinate) |
| | | | | 194.6 | 3.85 (H_{bz}) |
| IPA | Ambient | FFP | Good S_{A} | 2.8 + 4.6 (90.6) (2-step) | 1.02 (∼3H, IPA) |
| | | | | 155.8 | 2.32 (2H, succinate) |
| | | | | | 3.88 (H_{bz}) |
| Acetone | Ambient | FFP | Good B | 1.5 + 1.3 (2-step) | 2.31 (2H, succinate) |
| | | | | 162.3 | 3.86 (H_{bz}) |
| AcOEt | Ambient | FFP | Good B | 1.3 + 2.9 | - |
| | | | | 154.5 | |
| EtOH | 60 | SAM to FFP | Excellent A | - | - |
| EtOH:H₂O (1:0.025) | 60 | SAM to FFP | Excellent A | 1.0 | 2.31 (2H, succinate) |
| | | | | 197.3 | 3.85 (H_{bz}) |
| EtOH:H₂O (1:0.05) | 60 | SAM to FFP | Excellent A | - | - |

Four distinctly different hemi-succinate salts were isolated: a monohydrate (form A) (ethanol at ambient), a hemi-solvate of isopropanol (form S_{A}) (isopropyl alcohol), and two unsolvated forms A and B. Form A displays higher crystallinity, minimal weight loss up to 200°C, and higher decomposition temperature. In addition, it could be synthesized reproducibly, as demonstrated in ethanol and ethanol and water at 60°C.

### Reference EXAMPLE 17

### Formation of Hemi-Tartrate Salt

About 40-50 mg of N-hydroxy-3-[4-[[[2-(2-methyl-1H-indol-3-yl)ethyl]amino]methyl]phenyl]-2E-2-propenamide free base monohydrate was suspended in 1 mL of a solvent as listed in Table 17. A stoichiometric amount of tartaric acid was subsequently added to the suspension. The mixture was stirred at either 60°C or ambient temperature (where a clear solution formed, stirring continued at 4°C). Solids were collected by filtration and analyzed by XRPD, TGA and in some instances ¹H-NMR.

**Table 17**

| **Solvent** | **T, °C** | **Physical Appearance** | **Crystallinity and Form** | **LOD, % T_{decomposit.}** | **¹H-NMR** |
|---|---|---|---|---|---|
| EtOH:H₂O (1:0.05) | RT to 60 | FFP to SAM to FFP | Excellent A | 0.5 | 3.86 (1H, tartrate) |
| | | | | 206.9 | 3.95 (H_{bz}) |
| EtOH:H₂O (1:0.025) | 60 | SAM to FFP | Excellent A | - | - |
| EtOH:H₂O (1:0.05) | 60 | SAM to FFP | Excellent A | 0.5 | 3.86 (1H, tartrate) |
| | | | | 207.6 | 3.95 (H_{bz}) |
| EtOH | 60 | SAM to FFP | Excellent A | - | - |
| IPA:H₂O (1:0.05 | 60 | SAM to FFP | Good B | 1.9 and 3.4 > 160 °C | 3.90 (1H, tartrate) |
| | | | | | 3.96 (H_{bz}) |

The salt forming reaction of the free base with tartaric acid required heating to elevated temperatures. A highly crystalline, anhydrous salt that decomposed above 200°C was isolated as a hemi-tartrate and was labeled as form A. Form B was isolated once in isopropyl alcohol and water at 60°C and, although very similar in structure with A, significant differences were seen in its XRPD pattern.

### EXAMPLE 18

### Formation of Anhydrous DL-Lactate Salt

DL-lactic acid (4.0g, 85% solution in water, corresponding to 3.4 g pure DL-lactic acid) is diluted with water (27.2g), and the solution is heated to 90°C (inner temperature) for 15 hours. The solution is allowed to cool down to room temperature and is used as lactic acid solution for the following salt formation step.

N-hydroxy-3-[4-[[[2-(2-methyl-1H-indol-3-yl)ethyl]amino]methyl]phenyl]-2E-2-propenamide free base, form H_{A} (10.0 g) is placed in a 4-necked reaction flask with mechanical stirrer. Demineralized water (110.5 g) is added, and the suspension is heated to 65°C (inner temperature) within 30 minutes. The DL-lactic acid solution is added to this suspension during 30 minutes at 65°C. During the addition of the DL-lactic acid solution, the suspension converted into a solution. The addition funnel is rinsed with demineralized water (9.1 g), and the solution is stirred at 65°C for an additional 30 minutes. The solution is cooled down to 45°C (inner temperature) and seed crystals (10 mg N-hydroxy-3-[4-[[[2-(2-methyl-1H-indol-3-yl)ethyl]amino]methyl]phenyl]-2E-2-propenamide DL-lactate monohydrate) are added at this temperature. The suspension is cooled down to 33°C and is stirred for an additional 20 hours at this temperature. The suspension is re-heated to 65°C, stirred for 1 hour at this temperature and is cooled to 33°C within 1 hour. After additional stirring for 3 hours at 33°C, the product is isolated by filtration, and the filter cake is washed with demineralized water (2 x 20 g). The wet filter-cake is dried *in vacuo* at 50°C to obtain the anhydrous N-hydroxy-3-[4-[[[2-(2-methyl-1H-indol-3-yl)ethyl]amino]methyl]phenyl]-2E-2-propenamide DL-lactate salt as a crystalline product. The product is identical to the monohydrate salt (form H_{A}) in HPLC and in ¹H-NMR. XRPD indicated the presence of the anhydrate form.

In additional salt formation experiments carried out according to the procedure described above, the product solution was filtered at 65°C before cooling to 45°C, seeding and crystallization. In all cases, form A (anhydrate form) was obtained as product.

### EXAMPLE 19

### Formation of Anhydrous DL-Lactate Salt

DL-lactic acid (2.0g, 85% solution in water, corresponding to 1.7 g pure DL-lactic acid) is diluted with water (13.6 g), and the solution is heated to 90°C (inner temperature) for 15 hours. The solution was allowed to cool down to room temperature and is used as lactic acid solution for the following salt formation step.

N-hydroxy-3-[4-[[[2-(2-methyl-1H-indol-3-yl)ethyl]amino]methyl]phenyl]-2E-2-propenamide free base, Form H_{A} (5.0 g) is placed in a 4-necked reaction flask with mechanical stirrer. De-mineralized water (54.85 g) is added, and the suspension is heated to 48°C (inner temperature) within 30 minutes. The DL-lactic acid solution is added to this suspension during 30 minutes at 48°C. Seed crystals are added (as a suspension of 5 mg N-hydroxy-3-[4-[[[2-(2-methyl-1H-indol-3-yl)ethyl]amino]methyl]phenyl]-2E-2-propenamide DL-lactate salt, anhydrate form A, in 0.25 g of water) and stirring is continued for 2 additional hours at 48°C. The temperature is raised to 65°C (inner temperature) within 30 minutes, and the suspension is stirred for an additional 2.5 hours at this temperature. Then the temperature is cooled down to 48°C within 2 hours, and stirring is continued at this temperature for an additional 22 hours. The product is isolated by filtration, and the filter cake is washed with de-mineralized water (2 x 10 g). The wet filter-cake is dried *in vacuo* at 45-50°C to obtain anhydrous N-hydroxy-3-[4-[[[2-(2-methyl-1H-indol-3-yl)ethyl]amino]methyl]phenyl]-2E-2-propenamide DL-lactate salt (form A) as a crystalline product. Melting point and decomposition take place together at 183.3°C.

### EXAMPLE 20

### Conversion of DL-Lactate Salt Monohydrate to DL-Lactate Salt Anhydrate

DL-lactic acid (0.59 g, 85% solution in water, corresponding to 0.5 g pure DL-lactic acid) is diluted with water (4.1g), and the solution is heated to 90°C (inner temperature) for 15 hours. The solution is allowed to cool down to room temperature and is used as lactic acid solution for the following salt formation step.

10 g of N-hydroxy-3-[4-[[[2-(2-methyl-1H-indol-3-yl)ethyl]amino]methyl]phenyl]-2E-2-propenamide DL-lactate salt monohydrate is placed in a 4-necked reaction flask. Water (110.9 g) is added, followed by the addition of the lactic acid solution. The addition funnel of the lactic acid is rinsed with water (15.65 g). The suspension is heated to 82°C (inner temperature) to obtain a solution. The solution is stirred for 15 minutes at 82°C and is hot filtered into another reaction flask to obtain a clear solution. The temperature is cooled down to 50°C, and seed crystals are added (as a suspension of 10 mg N-hydroxy-3-[4-[[[2-(2-methyl-1H-indol-3-yl)ethyl]amino]methyl]phenyl]-2E-2-propenamide DL-lactate salt, anhydrate form, in 0.5 g of water). The temperature is cooled down to 33°C and stirring is continued for an additional 19 hours at this temperature. The formed suspension is heated again to 65°C (inner temperature) within 45 minutes, stirred at 65°C for 1 hour and cooled down to 33°C within 1 hour. After stirring at 33°C for an additional 3 hours, the product is isolated by filtration, and the wet filter cake is washed with water (50 g). The product is dried in vacuo at 50°C to obtain crystalline anhydrous N-hydroxy-3-[4-[[[2-(2-methyl-1H-indol-3-yl)ethyl]amino]methyl]phenyl]-2E-2-propenamide DL-lactate salt (form A).

### EXAMPLE 21

### Formation of Anhydrous DL-Lactate Salt

DL-lactic acid (8.0 g, 85% solution in water, corresponding to 6.8 g pure DL-lactic acid) was diluted with water (54.4 g), and the solution was heated to 90°C (inner temperature) for 15 hours. The solution was allowed to cool down to room temperature and was used as lactic acid solution for the following salt formation step.

N-hydroxy-3-[4-[[[2-(2-methyl-1H-indol-3-yl)ethyl]amino]methyl]phenyl]-2E-2-propenamide free base, Form H_{A} (20 g) is placed in a 1 L glass reactor, and ethanol/water (209.4 g of a 1:1 w/w mixture) is added. The light yellow suspension is heated to 60°C (inner temperature) within 30 minutes, and the lactic acid solution is added during 30 minutes at this temperature. The addition funnel is rinsed with water (10 g). The solution is cooled to 38°C within 2 hours, and seed crystals (20 mg of N-hydroxy-3-[4-[[[2-(2-methyl-1H-indol-3-yl)ethyl]amino]methyl]phenyl]-2E-2-propenamide DL-lactate salt, anhydrate form) are added at 38°C. After stirring at 38°C for an additional 2 hours, the mixture is cooled down to 25°C within 6 hours. Cooling is continued from 25°C to 10°C within 5 hours, from 10°C to 5°C within 4 hours and from 5°C to 2°C within 1 hour. The suspension is stirred for an additional 2 hours at 2°C, and the product is isolated by filtration. The wet filter cake is washed with water (2 x 30g), and the product is dried in vacuo at 45°C to obtain crystalline anhydrous N-hydroxy-3-[4-[[[2-(2-methyl-1H-indol-3-yl)ethyl]amino]methyl]phenyl]-2E-2-propenamide DL-lactate salt (form A).

### Reference EXAMPLE 22

### Formation of Sodium Salt

About 50 mg of N-hydroxy-3-[4-[[[2-(2-methyl-1H-indol-3-yl)ethyl]amino]methyl]phenyl]-2E-2-propenamide free base monohydrate was suspended in 1 mL of methanol. A stoichiometric amount of sodium hydroxide was subsequently added to the suspension. The mixture was stirred at 50°C. Once a clear solution formed, stirring continued at 4°C. Solids were collected by filtration and analyzed by XRPD and TGA. The sodium salt of N-hydroxy-3-[4-[[[2-(2-methyl-1H-indol-3-yl)ethyl]amino]methyl]phenyl]-2E-2-propenamide was isolated as a yellow highly hygroscopic powder, which absorbed moisture during filtration.

### Reference EXAMPLE 23

### Formation of Potassium Salt

About 50 mg of N-hydroxy-3-[4-[[[2-(2-methyl-1H-indol-3-yl)ethyl]amino]methyl]phenyl]-2E-2-propenamide free base monohydrate was suspended in 1 mL of methanol. A stoichiometric amount of potassium hydroxide was subsequently added to the suspension. The mixture was stirred at 50°C. Once a clear solution formed, stirring continued at 4°C. Solids were collected by filtration and analyzed by XRPD and TGA. The potassium salt of N-hydroxy-3-[4-[[[2-(2-methyl-1H-indol-3-yl)ethyl]amino]methyl]phenyl]-2E-2-propenamide was isolated as a yellow highly hygroscopic powder, which absorbed moisture during filtration.

### Reference EXAMPLE 24

### Formation of Calcium Salt

About 50 mg of N-hydroxy-3-[4-[[[2-(2-methyl-1H-indol-3-yl)ethyl]amino]methyl]phenyl]-2E-2-propenamide free base monohydrate was suspended in 1 mL of methanol. A stoichiometric amount of sodium hydroxide was subsequently added to the suspension. The mixture was stirred at 50°C. Once a clear solution formed, a stoichiometric amount of calcium dichloride was added causing an immediate precipitation of yellowish solid. Solids were collected by filtration and analyzed by XRPD and TGA. The calcium salt of N-hydroxy-3-[4-[[[2-(2-methyl-1H-indol-3-yl)ethyl]amino]methyl]phenyl]-2E-2-propenamide was less hygroscopic than the sodium or potassium salt of N-hydroxy-3-[4-[[[2-(2-methyl-1H-indol-3-yl)ethyl]amino]methyl]phenyl]-2E-2-propenamide and could be readily isolated.

### Reference EXAMPLE 25

### Formation of Zinc Salt

About 50 mg of N-hydroxy-3-[4-[[[2-(2-methyl-1H-indol-3-yl)ethyl]amino]methyl]phenyl]-2E-2-propenamide free base monohydrate was suspended in 1 mL of methanol. A stoichiometric amount of sodium hydroxide was subsequently added to the suspension. The mixture was stirred at 50°C. Once a clear solution formed, a stoichiometric amount of zinc sulfate was added causing an immediate precipitation of yellowish solid. Solids were collected by filtration and analyzed by XRPD and TGA. The zinc salt of N-hydroxy-3-[4-[[[2-(2-methyl-1H-indol-3-yl)ethyl]amino]methyl]phenyl]-2E-2-propenamide was less hygroscopic than the sodium or potassium salt of N-hydroxy-3-[4-[[[2-(2-methyl-1H-indol-3-yl)ethyl]amino]methyl]phenyl]-2E-2-propenamide and could be readily isolated.

### Reference EXAMPLE 26

### Formation of Hydrochloride Salt

3.67 g (10 mmol) of the free base monohydrate (N-hydroxy-3-[4-[[[2-(2-methyl-1H-indol-3-yl)ethyl]amino]methyl]phenyl]-2E-2-propenamide) and 40 mL of absolute ethanol were charged in a 250 mL 3-neck flask equipped with a magnetic stirrer and an addition funnel. To the stirred suspension were added dropwise 7.5 mL of 2 M HCl (15 mmol, 50% excess), affording a clear solution. A white solid precipitated out within 10 minutes, and stirring continued at ambient for an additional 2 hours. The mixture was cooled in an ice bath for approximately 30 minutes, and the white solid was recovered by filtration. It was washed once with cold ethanol (10 mL) and dried overnight under vacuum to yield 3.72 g of the chloride salt of N-hydroxy-3-[4-[[[2-(2-methyl-1H-indol-3-yl)ethyl]amino]methyl]phenyl]-2E-2-propenamide (96.2%).

It should be noted that HCl was used in excess to improve the yield, although equimolar amounts afforded yields of greater than 80%. Di-salt formation via protonation of the methyl-1H-indol-3-yl ring does not occur even when HCl is used in large excess. Reactions with 1, 1.5 and 2 equivalents of HCl afforded the same monochloride salt as a product. In addition, NMR data show no shifts for any of the protons in the vicinity of the ring, as it would have happened upon protonation.

### Reference EXAMPLE 27

### Formation of L-Tartarate Salt

3.67 g (10 mmol) of the free base monohydrate (N-hydroxy-3-[4-[[[2-(2-methyl-1H-indol-3-yl)ethyl]amino]methyl]phenyl]-2E-2-propenamide) and 50 mL of absolute ethanol were charged in a 250 mL 3-neck flask equipped with a magnetic stirrer and an addition funnel. The mixture was heated to 60°C, and to the hot suspension were added dropwise 0.83 g (5.5 mmol, 10% excess) of L-tartaric acid dissolved in 15 mL absolute ethanol. Initially, large yellow agglomerates formed that prevented adequate stirring, but overtime these were converted to free flowing and stirrable yellow powder. Stirring continued at 60°C for 2 hours. The mixture was subsequently cooled to room temperature and placed in an ice bath for approximately 30 minutes. The yellow powder was recovered by filtration and washed once by cold absolute ethanol (10 mL). It was dried overnight under vacuum to yield 4.1 g of the L-tartarate (hemi-tartarate) salt of N-hydroxy-3-[4-[[[2-(2-methyl-1H-indol-3-yl)ethyl]amino]methyl]phenyl]-2E-2-propenamide (96.6%).

### Reference EXAMPLE 28

### Formation of DL-Lactate Monohydrate Salt

3.67g (10 mmol) of the free base monohydrate, form H_{A} (N-hydroxy-3-[4-[[[2-(2-methyl-1H-indol-3-yl)ethyl]amino]methyl]phenyl]-2E-2-propenamide) and 75 mL of acetone were charged in a 250 mL 3-neck flask equipped with a magnetic stirrer and an addition funnel. To the stirred suspension were added dropwise 10 mL of 1 M lactic acid in water (10 mmol) dissolved in 20 mL acetone, affording a clear solution. Stirring continued at ambient and a white solid precipitated out after approximately 1 hour. The mixture was cooled in an ice bath and stirred for an additional hour. The white solid was recovered by filtration and washed once with cold acetone (15 mL). It was subsequently dried under vacuum to yield 3.94 g of the DL-lactate monohydrate salt of N-hydroxy-3-[4-[[[2-(2-methyl-1H-indol-3-yl)ethyl]amino]methyl]phenyl]-2E-2-propenamide (86.2%).

### Reference EXAMPLE 29

### Formation of Mesylate Salt

3.67 g (10 mmol) of the free base monohydrate (N-hydroxy-3-[4-[[[2-(2-methyl-1H-indol-3-yl)ethyl]amino]methyl]phenyl]-2E-2-propenamide) and 75 mL of ethyl acetate were charged in a 250 mL 3-neck flask equipped with a mechanical stirrer and an addition funnel. To the stirred suspension were added dropwise 0.65 mL (10 mmol) of methane sulfonic acid dissolved in 20 mL of ethyl acetate, affording a stirrable suspension of a free flowing yellow powder. The mixture was heated to 50°C and kept there overnight, and during that time the yellow powder converted to a white solid. The suspension was cooled to room temperature and the white solid was recovered by filtration. It was washed once with cold ethyl acetate (15 mL) and dried overnight under vacuum to yield 4.38 g of the mesylate salt of N-hydroxy-3-[4-[[[2-(2-methyl-1H-indol-3-yl)ethyl]amino]methyl]phenyl]-2E-2-propenamide (98.3%).

It is noted that the initially formed yellow powder is a polymorph of the mesylate salt that contains more than the equimolar amount of methane sulfonic acid. As a result, this solid is very highly hygroscopic. Upon gentle heating to 40°C or 50°C and within 2-4 hours, the yellow powder converts to a white crystalline solid that contains the equimolar amount of the methane sulfonic acid. This salt is non-hygroscopic. It is also advised that addition of the methane sulfonic acid is done at ambient temperature and the temperature increased afterwards. It was observed that addition at higher temperature afforded the immediate precipitation of the salt as a soft and gummy material.

### Reference EXAMPLE 30

### Formation of Maleate Salt

3.67 g (10 mmol) of the free base monohydrate (N-hydroxy-3-[4-[[[2-(2-methyl-1H-indol-3-yl)ethyl]amino]methyl]phenyl]-2E-2-propenamide) and 75 mL of acetone were charged in a 250 mL 3-neck flask equipped with a mechanical stirrer and an addition funnel. The mixture was heated to 45°C, and to the hot suspension were added dropwise 1.16 g (10 mmol) of maleic acid dissolved in 25 mL acetone. Although the addition was slow, the salt precipitated out as a soft gummy solid hindering stirring. Stirring continued overnight at 45°C and during that time the solid converted to a white free-flowing powder. The mixture was cooled to room temperature and placed in an ice bath for approximately 30 minutes. The white solid was recovered by filtration, washed once with cold acetone (15 mL), and dried overnight under vacuum to yield 4.21 g of the maleate salt of N-hydroxy-3-[4-[[[2-(2-methyl-1H-indol-3-yl)ethyl]amino]methyl]phenyl]-2E-2-propenamide (90.5%).

It is noted that a more preferable solvent for synthesis is 2-propanol. During optimization, however, it was observed that, in addition to the desired form, another polymorph with a low decomposition temperature (118.9°C) could be isolated from 2-propanol as a yellow powder.

### EXAMPLE 31

### Formation of Anhydrous L-(+)-Lactate Salt

N-hydroxy-3-[4-[[[2-(2-methyl-1H-indol-3-yl)ethyl]amino]methyl]phenyl]-2E-2-propenamide free base (20.0 g) was treated with L-(+)-lactic acid (6.8 g) according to the procedure described in Example 19 to obtain crystalline N-hydroxy-3-[4-[[[2-(2-methyl-1H-indol-3-yl)ethyl]amino]methyl]phenyl]-2E-2-propenamide L-(+)-lactate salt, anhydrate form. Melting point and decomposition take place together at 184.7°C. The XRPD pattern is as shown in Figure 3D (2θ = 9.9, 11.4, 13.8, 18.1, 18.5, 19.7, 20.2, 21.6, 25.2, 29.9).

### EXAMPLE 32

### Formation of Anhydrous D-(-)-Lactate Salt

N-hydroxy-3-[4-[[[2-(2-methyl-1H-indol-3-yl)ethyl]amino]methyl]phenyl]-2E-2-propenamide free base (20.0 g) was treated with D-(-)-lactic acid (6.8 g) according to the procedure described in Example 19 to obtain crystalline N-hydroxy-3-[4-[[[2-(2-methyl-1H-indol-3-yl)ethyl]amino]methyl]phenyl]-2E-2-propenamide D-(-)-lactate salt, anhydrate form. Melting point and decomposition take place together at 184.1°C. The XRPD pattern is as shown in Figure 3E (2θ = 9.9, 11.4, 13.8, 18.1, 18.5, 19.7, 20.2, 21.6, 25.2).

### Physical Characterization of Free Base, Hydrochloride, DL-Lactate, Maleate, Mesylate and Tartrate Salts

For each of the free base, chloride salt, maleate salt, mesylate salt and tartrate salts of N-hydroxy-3-[4-[[[2-(2-methyl-1H-indol-3-yl)ethyl]amino]methyl]phenyl]-2E-2-propenamide, a number of studies were conducted, namely to determine elemental composition, stoichiometry, purity, melting or decomposition point, pH of saturated solution, solubility, thermogravimetry, hygroscopic properties, intrinsic dissolution and stability.
HPLC method:
Instrument: Agilent 1100
Column: Zorbax SB-C18 (3.5µm), 150mm L x 3.0mm ID
Mobile phase: (A) 0.1% trifluoroacetic acid in water (v/v)
(B) 0.1% trifluoroacetic acid in acetonitrile (v/v)
Flow rate: 0.8 mL/min.
Column temp: 50°C
Gradient:

| **Time** | **% A** | **% B** |
|---|---|---|
| 0.00 | 97.0 | 3.0 |
| 2.00 | 97.0 | 3.0 |
| 15.00 | 77.0 | 23.0 |
| 25.00 | 55.0 | 45.0 |
| 27.00 | 55.0 | 45.0 |
| 27.01 | 97.0 | 3.0 |
| 35.00 | 97.0 | 3.0 |

Injection volume: 5 µL
Mass injected: 1 µg
Detection: UV, 280 nm
Sample solvent: Methanol

All samples were prepared/diluted to a concentration of 0.2 mg/mL in methanol prior to analysis by HPLC. A freshly prepared sample of each salt was used as the reference standard for external standard calibration analysis.

**LC/MS Analysis:**

| **Identity** | **RT (min.)** | **Mass (neutral)** | **Proposed structure** |
|---|---|---|---|
| Free base | 15.4 | 349 | 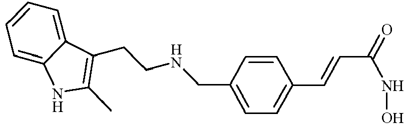 |
| Hydrolysis product | 16.3 | 334 | 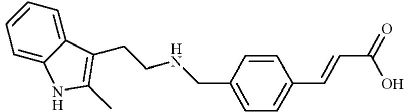 |
| By-product | 18.3 | 333 | 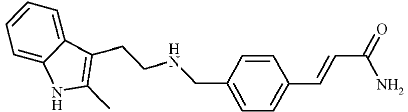 |
| Methylation | 25.0 | 348 | 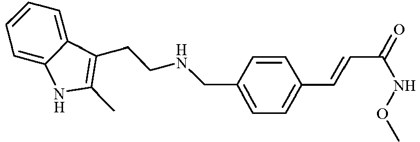 |

¹H-NMR spectra were recorded in DMSO-d₆.

DSC: All six substances decompose prior melting and therefore differential scanning calorimetry was not applicable.

pH Value: The pH at room temperature of a saturated solution or 1% suspension of the drug substance in water was recorded.

Aqueous Solubility: A carefully weighted amount (20-50 mg) of sample is dissolved in 1ml of solvent with 24-hour equilibration at room temperature. The solubility was determined either gravimetrically or by UV-VIS spectrometry. The pH of the clear solution was also measured. However, the difficulty of determining salt solubilities in water should be stressed, since upon dissolution dissociation to the free form is possible, which affects both the solubility and the pH. It's not unlikely that attempts to make solutions of a salt at a concentration well below the reported solubility of the salt to be unsuccessful (for a full discussion see: M. Pudipeddi, A. T. M. Serajuddin, D. J. W. Grant, and P. H. Stahl in "Handbook of Pharmaceutical Salts Properties Selection and Use" page 27 and references therein).

Clear solutions of the mesylate salt at concentration below the reported solubility could be made initially, but over time of storage solid precipitation occurred. In addition, a polymorphic transformation was observed for the mesylate salt in aqueous solutions. The residue in both cases was analyzed by mass spec and found to be the free base, indicating that the precipitate is not a decomposition product.

Intrinsic Dissolution: Approximately 30 mg of each substance were pressed to pellets of 0.13 cm². Most of the free base pellet disintegrated upon contact with the aqueous dissolution media, and thus the dissolution rate reported above does not correspond to the true intrinsic dissolution of the free base. In 0.1 N HCl the free base pellet disintegrated completely and the dissolution rate was not determined. Pellets of the other salts remained intact for at least several minutes enabling the determination of the intrinsic dissolution rate. Dissolution rate studies were performed using the rotation disk method (VanKell Instrument). A single rotation speed of 200 r.p.m. was used to dissolve drug substance into a 500 mL vessel at 37°C. The solution was continuously pumped through a UV cell measurement and returned to the dissolution vessel.

The results for the above-noted studies are presented in Table 18 below.

**Table 18**

| | **Salt Form** | | | | | | | | | |
|---|---|---|---|---|---|---|---|---|---|---|
| **Elemental analysis** | **Free base** | | **HCl** | | **L-Tartrate** | | **Mesylate** | | **Maleate** | |
| | Calc | Fnd | Calc | Fnd | Calc | Fnd | Calc | Fnd | Calc | Fnd |
| % C | 68.64 | 68.53 | 65.36 | 65.09 | 65.08 | 6524 | 59.31 | 59.13 | 64.51 | 64.19 |
| % H | 6.86 | 6.74 | 627 | 6.64 | 6.17 | 6.36 | 6.11 | 6.12 | 5.85 | 5.65 |
| % N | 11.44 | 11.41 | 10.89 | 10.77 | 9.90 | 9.94 | 9.43 | 9.39 | 9.03 | 8.92 |
| % S | | | - | - | | | 720 | 7.26 | | |
| % Cl | | | 9.19 | 9.06 | | | | | | |
| Stoichiometry | | | | | | | | | | |
| ¹H-NMR | NA | | 1:1 | | 2:1 | | 1:1 | | 1:1 | |
| DSC-Puritv | | | | | | | | | | |
| Heating rate 2°C/min | Not applicable | | Not applicable | | Not applicable | | Not applicable | | Not applicable | |
| HPLC-Purity (e.g. area-%) | | | | | | | | | | |
| | 99.41 | | 99.63 | | 99.62 | | 99.30 | | 99.48 | |
| Melting Point (DSC) | | | | | | | | | | |
| Heating rate [10 K/min] in °C | Not applicable | | Not applicable | | Not applicable | | Not applicable | | Not applicable | |
| Melting enthalpv (J/g) | Not applicable | | Not applicable | | Not applicable | | Not applicable | | Not applicable | |
| pH of saturated solution | | | | | | | | | | |
| In water | 8.7 | | 5.65 | | 6.07 | | 4.34 | | 5.54 | |
| In pH = 6.8 buffer | 6.91 | | 5.67 | | 5.57 | | 5.38 | | 5.70 | |
| Solubility (approx. at 25°C, mg/mL) | | | | | | | | | | |
| Methanol | 2.3 | | 16.6 | | 2.6 | | > 115 | | 57.0 | |
| Ethanol | 1.5 | | 2.1 | | 0.5 < | | 14.6 | | 7.2 | |
| 2-Propanol | 4.0 | | 0.8 | | 0.3 < | | 2.2 | | 1.6 | |
| Acetone | 6.5 | | 4.5 | | 4.6 | | 3.0 | | 3.0 | |
| Ethyl acetate | 5.6 | | 6.5 | | 3.9 | | 6.4 | | 5.6 | |
| Water | 0.004 | | 2.4 | | 3.5 | | 12.9 | | 2.6 | |
| 0.1 N HCl | 0.3 | | 0.2 | | 0.4 | | 0.6 | | 0.7 | |
| pH = 6.8 buffer | 0.3 | | 0.7 | | 1.9 | | 4.1 | | 1.5 | |
| Propylene glycol | 4.9 | | 13.2 | | 7.2 | | 46.5 | | 32.4 | |
| Thermogravimetry (weight loss in%) | | | | | | | | | | |
| LOD in % | 4.8% | | 0.4% | | 0.3% | | 0.2% | | 0.1% | |
| Tonset dehydration temperature | Ambient | | - | | - | | - | | - | |
| Tonset decomposition temperature | 157.4 °C | | 235.7 °C | | 209.0 °C | | 192.4 °C | | 176.7 °C | |
| Intrinsic Dissolution Rate (mg min⁻¹ cm⁻²) | | | | | | | | | | |
| HCl 0.1 N | NA | | 0.13 | | 1.16 | | 6.51 | | 1.00 | |
| Water | 0.15 | | 0.68 | | 0.38 | | 10.17 | | 0.32 | |

As can be seen from Table 18, each of the salts outperforms the solubility of the free base by approximately 3 orders of magnitude. The hydrochloride, maleate and L-tartrate salts have very similar solubilities at approximately 0.3%. The mesylate salt is the most soluble of all at 1.3%. (Approximate solubilities were estimated from the concentration in mg/mL, assuming that the density of a solution is 1 g/mL.) Intrinsic dissolution rates varied accordingly.

In addition, for each of the monohydrate DL-lactate salt and the anhydrous DL-lactate salt, a number of studies were conducted, namely to determine purity, melting or decomposition point, thermogravimetry, hygroscopic properties and intrinsic dissolution. The results of those studies are set forth in Table 19 below.

**Table 19**

| | **Monohydrate DL-lactate salt** | **Anhydrous DL-lactate salt** |
|---|---|---|
| Purity (HPLC) | 98.4% | NA |
| DSC melting onset | 111C | 181C |
| Thermogravimetry (TG, 10K/min) | 2.7% (up to 130°C) | 0.39% (up to 130°C) |
| Water content (Karl Fischer) | 4.3% | 0.69% |
| Hygroscopicity (DVS) | Slight 0.55% at 80% r.h. | Slight 0.69% at 80% r.h. |
| Intrinsic dissolution rate | | |
| 0.1 N HCl | 0.02 | 0.13 |
| pH=4 | 0.08 | 0.09 |
| Water | 0.06 | 0.14 |

Also stirring experiments were conducted with respect to the monohydrate and anhydrous DL-lactate salts. In particular, a mixture of forms A and H_{A} of the DL-lactate salt were stirred over certain times and temperatures. The results of those experiments are set forth in Tables 20 and 21 below.

**Table 20**

| Temperature (°C)/stirring time | 2 | 10 | 20 | 25 | 30 |
|---|---|---|---|---|---|
| 2 days | No change | No change | No change | No change | No change |
| 8 days | No change | No change | Increase of A | Conversion to A | Conversion to A |
| 24 days | Increase of A | Increase of A | Conversion to A | Conversion to A | Conversion to A |

**Table 21**

| Temperature (°C) | 25 | 35 | 50 | 70 |
|---|---|---|---|---|
| After 24 hours | No change | Increase of A | Conversion to A | Conversion to A |

The stability of each of the free base, hydrochloride salt, maleate salt, monohydrate DL-lactate salt, mesylate salt and hemi-tartrate salts of N-hydroxy-3-[4-[[[2-(2-methyl-1H-indol-3-yl)ethyl]amino]methyl]phenyl]-2E-2-propenamide in solution (Table 22), in solid state (Table 23) and in the presence of excipient mixtures (Table 24) was also determined.

**Table 22 Solution Stability**

| | **Salt Form** | | | | | | | | | | | |
|---|---|---|---|---|---|---|---|---|---|---|---|---|
| | Base | | Mesylate | | Tartrate | | Hydrochloride | | DL-Lactate Monohydrate | | Maleate | |
| Unstressed (% Area) | 99.41% | | 99.30% | | 99.62% | | 99.63% | | 99.51% | | 99. | 48% |
| | % DP [assay] | CL | % DP [assay] | CL | % DP [assay] | CL | % DP [assay] | CL | % DP [assay] | CL | % DP [assay] | CL |
| 2 mg/mL solutions/suspensions w/100 mM lactate buffer, pH 3.5 for 1 week at 50°C | | | | | | | | | | | | |
| pH of initial mixture | 3.60 | | 3.48 | | 3.58 | | 3.52 | | 3.57 | | 3.51 | |
| Stability results | 1.47 [97.21] | A | 1.60 [99.25] | A | 1.53 [96.89] | A↓* | 1.30 [96.61] | A↓* | 1.31 [99.12] | A↓* | 1.59 [97.48] | A↓* |
| 2 mg/mL solutions/suspensions in water for 1 week at 50°C | | | | | | | | | | | | |
| pH of initial mixture | 9.59 | | 6.55 | | 6.82 | | 5.93 | | 6.30 | | 5.40 | |
| Stability results | 0.73 [98.73] | A↓ | 1.16 [99.30] | A | 1.21 [98.91] | A↓ | 0.89 [97.53] | A↓* | 1.22 [98.85] | A↓ | 0.80 [97.26] | A↓ |
| 2 mg/mL solutions/suspensions in methanol for 1 week at 50°C | | | | | | | | | | | | |
| Stability results | 1.50 [100.2] | A | 0.62 [101.2] | A | 0.86 [100.5] | A↓ | 0.38 [99.25] | A | 0.71 [102.8] | A | 0.83 [100.7] | A |
| 2% solutions/suspensions, 1 day at RT | | | | | | | | | | | | |
| 0.5% CMC | 0.64 [98.07] | A | 0.74 [79.72] | A↓↓ | 0.62 [79.98] | A↓↓ | 0.46 [85.28] | A↓↓ | 0.49 [77.50] | A↓↓ | 0.58 [77.42] | A↓↓ |
| 0.5% HPMC 4000 | 0.65 [100.4] | A | 0.66 [97.30] | A | 0.54 [97.54] | A | 0.43 [100.0] | A | 0.41 [96.42] | A | 0.60 [94.63] | A |
| 0.5% Klucel HF | 0.65 [99.28] | A | 0.66 [97.63] | A | 0.58 [96.39] | A | 0.43 [100.6] | A | 0.43 [96.99] | A | 0.55 [96.78] | A |
| 0.8% Tween 80 | 0.67 [98.91] | A | 0.63 [98.05] | A | 0.54 [96.60] | A | 0.43 [97.83] | A | 0.42 [95.06] | A | 0.66 [96.83] | A |
| 5% solution, 1 day at RT (diluted 1:100 in pH 6.8 buffer) | | | | | | | | | | | | |
| Stability results | 0.69 [100.4] | A↓ | 0.78 [99.30] | A | 0.71 [98.39] | A↓ | 1.40 [98.91 | A↓ | 0.52 [99.24] | A↓ | 0.67 [97.92] | A↓ |

**Table 23 Solid-State Stability**

| | **Salt Form** | | | | | | | | | | | |
|---|---|---|---|---|---|---|---|---|---|---|---|---|
| | Base | | Mesylate | | Tartrate | | Hydrochloride | | DL-lactate Monohydrate | | Maleate | |
| | % DP [assay] | CL | % DP [assay] | CL | % DP [assay] | CL | % DP [assay] | CL | % DP [assay ] | CL | % DP [assay] | CL |
| Bulk stability, 2 weeks | | | | | | | | | | | | |
| 50°C | 0.71 [97.76] | A | 0.80 [99.35] | A | 0.84 [99.48] | A | 0.44 [99.38] | A | 0.43 [99.31] | A | 0.71 [102.2] | A |
| 50°C/75% r.h. | 0.66 [98.61] | A | 1.20 [100.0] | A | 1.36 [97.66] | A | 0.56 [99.04] | A | 0.43 [99.86] | A | 0.58 [100.0] | A |
| 50°C/20% water | 0.72 [99.48] | A | 1.48 [97.36] | A | 1.61 [97.07] | A | 0.56 [100.6] | A | 0.51 [100.3] | A | 0.71 [101.1] | A |
| Light study | | | | | | | | | | | | |
| 1200 kLux (300 - 800nm) | 2.22 [99.39] | B | 1.16 [98.28] | B | 0.82 [100.4] | A | 2.58 [98.15] | C | 1.68 [98.65] | C | 3.02 [96.72] | D |
| Bulk stability, 1 week (XRPD) | | | | | | | | | | | | |
| 80°C | Changed to mod.C | | No change | | no change | | no change | | no change | | no change | |
| 80°C/75% r.h. | no change | | No change | | no change | | no change | | no change | | no change | |
| Corrosivity | | | | | | | | | | | | |
| Appearance | A | | A | | A | | A | | A | | A | |

| | | | | | | | | | | | | |
|---|---|---|---|---|---|---|---|---|---|---|---|---|
| 1. DP = total degradation products (% area). The total % degradation products (DP) and assay may not total 100% since the relative response factors of the unknown impurities have not been determined. 2. Assay is determined by external standard analysis compared to a freshly prepared standard of the corresponding salt. 3. = suspension; * = clear solution after stress test; ↓↓ = could not be completely dissolved in sample solvent after stress test. 4. Appearance: A = no change, B = slight discoloration, C = medium discoloration, D = strong discoloration | | | | | | | | | | | | |

**Table 24 Stability of Mixtures**

| | **Salt Form** | | | | | | | | | | | |
|---|---|---|---|---|---|---|---|---|---|---|---|---|
| | Base | | Mesylate | | Tartrate | | Hydrochloride | | DL-lactate Monohydrate | | Maleate | |
| | %DS | %DP | %DS | %DP | %DS | %DP | %DS | %DP | %DS | %DP | %DS | %DP |
| Stability at 50°C/75% r.h., 2 weeks | | | | | | | | | | | | |
| Standard | 99.5 | 0.5 | 99.5 | 0.5 | 99.4 | 0.6 | 99.7 | 0.3 | 99.6 | 0.4 | 99.8 | 0.2 |
| Mixture 1 | 98.8 | 1.2 | 99.2 | 0.8 | 98.8 | 1.2 | 99.4 | 0.6 | 99.4 | 0.6 | 99.5 | 0.5 |
| Mixture 2 | 99.4 | 0.6 | 99.3 | 0.7 | 98.7 | 1.3 | 99.5 | 0.5 | 99.6 | 0.4 | 99.7 | 0.3 |
| Mixture 3 | 99.3 | 0.6 | 99.3 | 0.7 | 98.7 | 1.3 | 99.4 | 0.6 | 99.5 | 0.5 | 99.7 | 0.3 |
| Mixture 4 | 99.4 | 0.5 | 99.2 | 0.8 | 98.7 | 1.3 | 99.5 | 0.5 | 99.5 | 0.5 | 99.7 | 0.3 |
| Mixture 5 | 99.2 | 0.8 | 99.2 | 0.8 | 98.7 | 1.3 | 99.5 | 0.5 | 99.5 | 0.5 | 99.7 | 0.3 |
| Mixture 6 | 99.4 | 0.6 | 99.3 | 0.7 | 98.7 | 1.3 | 99.5 | 0.5 | 99.3 | 0.6 | 99.7 | 0.3 |
| Mixture 7 | 99.5 | 0.5 | 99.4 | 0.6 | 98.7 | 1.3 | 99.5 | 0.5 | 99.5 | 0.5 | 99.7 | 0.3 |
| Mixture 8 | 98.6 | 0.6 | - | - | 98.7 | 1.3 | 99.5 | 0.5 | 99.4 | 0.6 | 99.6 | 0.4 |

| | | | | | | | | | | | | |
|---|---|---|---|---|---|---|---|---|---|---|---|---|
| 1. The standard used was freshly prepared; 2. DP = total degradation products (% area) and DS = drug substance (% area); 3. - = not performed Mixture 1: 50% PVP + 50% Crospovidone Mixture 2: 50% Starch 1500 + 50% MCC 102 Mixture 3: 5% PVP + 5% Crospovidone + 10% Starch 1500 + 80% MCC 102 Mixture 4: 99% Lactose + 1% BHT/BHA Mixture 5: 99% Mannitol + 1 % BHT/BHA Mixture 6: 50% Mannitol + 47% HPCLH21 + 1% BHT/BHA + 2% Magnesium Stearate (*Note*: 1% Magnesium Stearate mixed w/ salt first) Mixture 7: 50% Cetyl Alcohol + 49% HPCLH21 + 1% Magnesium Stearate Mixture 8: 100% PEG 3350 | | | | | | | | | | | | |

Each of the free base, hydrochloride salt, DL-lactate salt, maleate salt, mesylate salt and tartrate salts of N-hydroxy-3-[4-[[[2-(2-methyl-1H-indol-3-yl)ethyl]amino]methyl]phenyl]-2E-2-propenamide exhibited very good stability characteristics both in solution and in the solid state. Approximately, 1.5% total degradation was observed for all salts and free base as solutions in lactate buffer (pH 3.5), water, and methanol. The salts also exhibited very good stability in all tox solutions tested (CMC, HPMC, Klucel and Tween-80).

In addition, each of the free base, hydrochloride salt, DL-lactate salt, maleate salt, mesylate salt and tartrate salts of N-hydroxy-3-[4-[[[2-(2-methyl-1H-indol-3-yl)ethyl]amino]methyl]phenyl]-2E-2-propenamide also exhibited very good stability with all excipient mixtures tested after 2 weeks at 50°C/75% r.h.

### Supplemental Testing

An approximate solubility of the below-listed salts was determined in water and at pH 1 by suspending 5-15 mg of the salt in 1 mL of solvent. The samples were allowed to equilibrate at ambient temperature for at least 20 hours. The supernatant was filtered and used for the solubility determination, which was done gravimetrically, for the aqueous solubility, and by UV-VIS spectroscopy for pH 1. The solid residue was analyzed by XRPD. Additionally, solid samples of the below-listed salts were held at 93% r.h. for either 7 or 10 days. They were subsequently analyzed by XRPD and TGA, if the latter deemed necessary. Only irreversible or slowly reversible events can be detected. Results are listed in Table 25 below.

**Table 25**

| **Salt (Base:SFA Ratio)** | **Solution (EQ t > 20 hours)** | | | | **Solid State (EQ 93% r.h.)** |
|---|---|---|---|---|---|
| | Water | | pH 1 | | |
| | S mg/mL | Crystallinity & Form by XRPD | S mg/mL | XRPD Pattern | Crystallinity & Form by XRPD LOD by TGA |
| Acetate (1:1) | 2.18 | Good B (new form) | 0.27 | Corresponds to hydrochloride salt | Good B LOD=8.8% (105°C) |
| Benzoate (1:1) | 0.69 | Excellent B (new form) | 0.50 | Corresponds to hydrochloride salt | No change (7 days) |
| Citrate | 1.25 | No change | 0.28 | Corresponds to hydrochloride salt | No change (10 days) |
| Fumarate (2:1) | 0.41 | Excellent C (new form) | 0.35 | Corresponds to hydrochloride salt | No change (10 days) |
| Gentisate | 0.25 | No change | 0.30 | Corresponds to hydrochloride salt | No change (10 days) |
| Malate | 1.38 | No change | 0.42 | Corresponds to hydrochloride salt | No change (10 days) |
| Malonate | 1.92 | Amorphous | 0.49 | Corresponds to hydrochloride salt | No change (10 days) |
| Propionate | 4.19 | NA free base residue | 0.34 | Corresponds to hydrochloride salt | Poor crystallinity |
| Phosphate | 6.26 | (poor crystallinity) no change | 0.61 | Corresponds to hydrochloride salt | No change (7 days) |
| Succinate | 0.39 | Excellent C (new form) | 0.29 | Corresponds to hydrochloride salt | No change (10 days) |

As can be seen in Table 25 above, most salts did not undergo any irreversible transformation upon storage at 93% RH for either 7 or 10 days. However, the following observations were noted: acetate converted to a new form, which was also isolated upon the equilibration of the salt in water. It is likely that this new form constitutes a hydrate.

The solid residues from the equilibration in water were examined by XRPD and other techniques when deemed necessary. The results can be summarized as follows:
- No structural change was observed in the salts of citrate, gentisate, malate and phosphate.
- The solid residue of the propionate equilibration consisted of the free base only.
- Acetate, benzoate, fumarate and succinate converted to new salt polymorphs.

In view of the fact that XRPD analysis showed that in all cases, with the exception of the propionate salt, the solution was in equilibrium with the corresponding salt, the aqueous solubilities in Table 25 are representative of the salt (Chapter 2, in Handbook of Pharmaceuticals Salts; Authors: M. Pudipeddi, A. T. M. Serajuddin, D. J. W. Grant, and P. H. Stahl).

During equilibration in pH 1 buffer solutions, all the salts converted to the chloride salt. This is reflected in the narrow range of the solubilities observed, which all lie between 0.3 and 0.6 mg/ml (S = 0.25 mg/mL for chloride salt).

## Claims

1. An anhydrous lactate salt of N-hydroxy-3-[4-[[[2-(2-methyl-1H-indol-3-yl)ethyl]amino]methyl]phenyl]-2E-2-propenamide.

2. The salt of Claim 1, wherein the lactate salt is a 1:1 DL-lactate salt.

3. The salt of Claim 1, wherein the lactate salt is an L-lactate salt.

4. The salt of Claim 1, wherein the lactate salt is a D-lactate salt.

5. A pharmaceutical composition comprising:
(a) a therapeutically effective amount of a salt according to any one of Claims 1-4;
and
(b) at least one pharmaceutically acceptable carrier, diluent, vehicle or excipient.

## Patentansprüche

1. Wasserfreies Lactatsalz von N-Hydroxy-3-[4-[[[2-(2-methyl-1H-indol-3-yl)ethyl]amino]methyl]phenyl]-2E-2-propenamid.

2. Salz nach Anspruch 1, wobei das Lactatsalz ein 1:1-DL-Lactatsalz ist.

3. Salz nach Anspruch 1, wobei das Lactatsalz ein L-Lactatsalz ist.

4. Salz nach Anspruch 1, wobei das Lactatsalz ein D-Lactatsalz ist.

5. Pharmazeutische Zusammensetzung, umfassend:
(a) eine therapeutisch wirksame Menge eines Salzes nach einem der Ansprüche 1-4;
und
(b) mindestens einen pharmazeutisch annehmbaren Träger, ein pharmazeutisch annehmbares Verdünnungsmittel, Vehikel oder einen pharmazeutisch annehmbaren Hilfsstoff.

## Revendications

1. Sel de lactate anhydre de N-hydroxy-3-[4-[[[2-(2-méthyl-1H-indol-3-yl)éthyl]amino]méthyl]phényl]-2E-2-propénamide.

2. Sel selon la revendication 1, dans lequel le sel de lactate est un sel de DL-lactate 1:1.

3. Sel selon la revendication 1, dans lequel le sel de lactate est un sel de L-lactate.

4. Sel selon la revendication 1, dans lequel le sel de lactate est un sel de D-lactate.

5. Composition pharmaceutique comprenant :
(a) une quantité thérapeutiquement efficace d'un sel selon l'une quelconque des revendications 1 à 4 ;
et
(b) au moins un support, diluent, véhicule ou excipient pharmaceutiquement acceptable.
